# EUROPEAN PATENT SPECIFICATION

(11) **EP 0 266 730 B1**
(45) Date of publication and mention of the grant of the patent: **10.08.1994**
(21) Application number: 87116119.6
(22) Date of filing: 02.11.1987
(51) Int. Cl.: C07D 455/00, C07D 471/18, A61K 31/445

(54) **Esters of hexadydro-8-hydroxy-2,6-methano-2H-quinolizin-3(4H)-one and related compounds**
Ester des Hexahydro-8-hydroxy-2,6-methano-2H-chinolizin-3(4H)-ons und verwandte Verbindungen
Esters de l'hexahydro-8-hydroxy-2,6-méthano-2H-quinolizin-3-(4H)-one et composés apparentés

(30) Priority: 03.11.1986 US 926619
(43) Date of publication of application: 11.05.1988
(73) Proprietor: MERRELL DOW PHARMACEUTICALS INC., Cincinnati Ohio 45215-6300 (US)
(72) Inventor: Gittos, Maurice Ward, F-67115 Plobsheim (FR)
(74) Representative: VOSSIUS & PARTNER

(56) References cited:
- EP-A- 189 002
- WO-A-84/00166
- WO-A-85/01048
- NATURE (1985) 316, 126-131
- ACTUALITES DE CHIMIE THERAPEUTIQUE (1989) 16, 187-198
- ARCHIV DER PHARMAZIE, vol. 309, 1976, W. SCHNEIDER et al.: "Beiträge zur Tropanchemie, 2. Mitt. Nortropan-3B-Essisäure, Tropachinuclidin und Dehydrotropachinuclidin", pages 447-457.
- TETRAHEDRON LETERS, vol. 15, 1966; W. SCHNEIDER et al.:"9-Aza-Tricyclo[4.3.1.0.4,9]decanon-(7) ("Tropachinuclidon")", pages 1583-1586.
- ARCHIV DER PHARMAZIE, vol. 308, 1975; W. SCHNEIDER et al.:"Beitäge zur Tropanchemie, 1. Mitt. 3B-substituierte Tropan-Derivate", pages 365-375.
- JOURNAL OF THE CHEMICAL SOCIETY CHEMICAL COMMUNICATION, 1976; P.G. SAMMES et al.:"Novel Heterocyclic Systems by intramolecular cycloadditions with unactivated olefins", pages 367-368.
- HELVETICA CHIMICA ACTA, vol. 59, 1976; B. MAURER et al.:"Zur Kenntnis der stickstoffhaltigen Inhaltsstoffe von Castoreum", pages 1169-1185.

## Description

The present invention is directed to esters of hexahydro-8-hydroxy-2,6-methano-2H-quinolizin-3-(4H)-one and hexahydro-8-hydroxy-2,6-methano-2H-quinolizines with certain aromatic and heterocyclic carboxylic acids. The invention is also directed to novel polycyclic alcohols which serve as intermediates in the preparation of the esters of this invention and also to a novel process for preparing a group of esters of the present invention.

More particularly, the present invention is directed to compounds of the formula 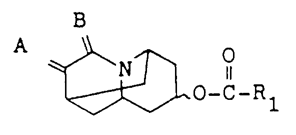
wherein A is =H₂, =O, =(H)(OH), or =N-OH; B is =H₂, =(H)(CH₃), =(H)(CH₂NR₃R₄) or =CH₂ wherein R₃ and R₄ are C₂₋₄ alkyl or are combined to give tetramethylene, pentamethylene or -CH₂CH₂-O-CH₂CH₂-; R₁ is 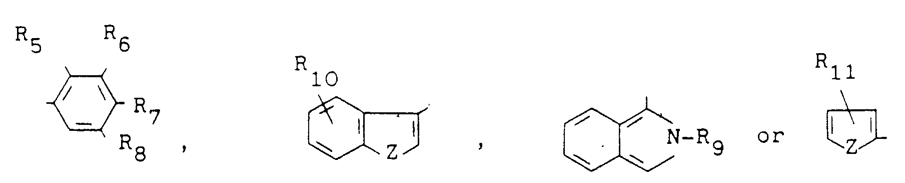
wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; the wavy line indicates that the configuration of the oxygen substituent on the ring can be endo or exo; and the pharmaceutically acceptable acid addition and quaternary ammonium salts of the aforesaid compounds.

Examples of the C₁₋₄ alkyl groups referred to above are methyl, ethyl, propyl, isopropyl and butyl. Examples of the C₁₋₄ alkoxy groups are methoxy, ethoxy, propoxy and butoxy. The halogens referred to above can be fluorine, chlorine or bromine. When the wavy line in the general structural formula is changed to a solid line, this indicates that the configuration of the compounds is endo. Such endo-compounds can also be referred to as trans. Similarly, exo-compounds can also be referred to as cis. Any hydrates of the present compounds are considered as equivalent to the compounds themselves and this would include compounds in which the carbonyl (i.e., A is O) exists as (OH)₂.

A preferred group of compounds are those wherein the ester is attached to the polycyclic ring in the endo-configuration. A further preferred group are those having the endo-configuration wherein A is =O and =(OH)₂. In a still further preferred group, B is additionally =H₂.

The pharmaceutically acceptable acid addition salts referred to above can be non-toxic salts with suitable acids such as those with inorganic acids, for example hydrochloric, hydrobromic, nitric, sulfuric or phosphoric acids; or with organic acids such as organic carboxylic acids, for example, acetic, propionic, glycolic, maleic, hydroxymaleic, malic, tartaric, citric, salicyclic, 2-acetyloxybenzoic, nicotinic or isonicotinic; or organic sulfonic acids, for example methanesulfonic, ethane-sulfonic, 2-hydroxyethanesulfonic, 4-toluenesulfonic or 2-naphthalensulfonic. Quaternary ammonium salts are formed with alkyl halides such as methyl chloride, methyl bromide or ethyl bromide; or with sulfate esters such as methyl 4-toluenesulfonate or methyl 2-naphthalenesulfonate.

Some specific examples of compounds encompassed by the present invention are the following:
endo-8-(3,5-Dimethylbenzoyloxy)hexahydro-2,6-methano-2H-quinolizin-3(4H)-one
exo-8-(3,5-Dimethylbenzoyloxy)hexahydro-2,6-methano-2H-quinolizin-3(4H)-one
endo-8-(3,5-Dichlorobenzoyloxy)hexahydro-2,6-methano-2H-quinolizin-3(4H)-one
endo-8-(3,5-Dimethoxybenzoyloxy)hexahydro-2,6-methano-2H-quinolizin-3(4H)-one
endo-8-(4-Aminobenzoyloxy)hexahydro-2,6-methano-2H-quinolizin-3(4H)-one
endo-8-(4-Dimethylaminobenzoyloxy)hexahydro-2,6-methano-2H-quinolizin-3(4H)-one
endo-8-(3,5-Dimethylbenzoyloxy)octahydro-2,6-methano-2H-quinolizine
endo-8-(3-Indolylcarbonyloxy)octahydro-2,6-methano-2H-quinolizine
endo-8-(5-Cyano-3-indolylcarbonyloxy)hexahydro-2,6-methano-2H-quinolizin-3(4H)-one
endo-8-(3,5-Dichlorobenzoyloxy)hexahydro-2,6-methano-4-methyl-2H-quinolizin-3(4H)-one
endo-8-(3-Indolylcarbonyloxy)hexahydro-4-(diethylaminomethyl)-2,6-methano-2H-quinolizin-3(4H)-one
endo-8-(3-Indolylcarbonyloxy)-3-hydroxyimino-2,6-methanooctahydro-2H-quinolizine
endo-8-(2-Methyl-1-isoindolylcarbonyloxy)hexahydro-2,6-methano-2H-quinolizin-3(4H)-one
endo-8-(2-Pyrrolidinylcarbonyloxy)hexahydro-2,6-methano-2H-quinolizin-3(4H)-one
endo-8-(3-Indolycarbonyloxy)-2,6-methanooctahydro-2H-quinolizin-3-ol
The compounds of the present invention can be prepared by reacting an alcohol or a reactive derivative thereof, said alcohol having the formula 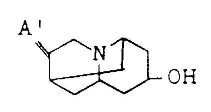
wherein A' is =O or =H₂, with a reactive equivalent of an acid of the formula

R₁COOH

wherein R₁ is defined as above. By a reactive equivalent of the acid is meant the corresponding acid chloride or bromide or the corresponding glyoxylyl chloride or bromide or the carboxylic acid imidazole obtained by the reaction of the appropriate acid halide with N,N-carbonyldiimidazole; or any similar acid derivative which would yield the simple carboxylic acid ester on reaction with an alcohol or with a reactive derivative of an alcohol. More specifically, where the -OH in the alcohol is equatorial (exo), then it can be reacted with the appropriate carboxylic acid imidazole obtained by the reaction of the acid halide with N,N-carbonyldiimidazole. Alternatively, the acid car be converted to the acid chloride by standard procedures (e.g., thionyl chloride) and then reacted with the alcohol or an alkali metal salt of the alcohol such as the lithium salt obtained by the reaction of lithium hydride with the alcohol in tetrahydrofuran.

When the -OH group in the starting alcohol is axial (endo), it can also be converted to the corresponding ester by reaction with the appropriate acid chloride or bromide with the reaction being carried out in the presence of an equivalent of a suitable tertiary base such as 4-dimethylaminopyridine in a high boiling inert solvent such as xylene. In this case, however, long heating (24-84 hours) at a temperature at or above 140°C is necessary so that the procedure would not be suitable for use with acid halides that are not stable under the indicated conditions. Thus, it was necessary to use an alternative for the preparation of such compounds. In this procedure, an appropriate acid chloride or bromide or a glyoxylyl chloride or bromide, in a nitroparaffin solvent, is reacted with a solution of a super acid salt of the alcohol and an equivalent amount of a heavy metal salt of the same super acid. The glyoxylyl chloride can be used in the process as indicated because it decarbonylates readily under the conditions used. The reaction itself can be carried out over a period of 1-24 hours at temperatures ranging from -80°C to ambient temperatures (about 23°C). Examples of suitable super acids with M = H are MBF₄, MAsF₆, MSbF₆, MPF₆, MTaF₆ or MNbF₆ with examples of suitable heavy metals (M) being silver and thallium. Examples of nitroparaffin solvents are nitromethane, nitroethane, 1-nitropropane and 2-nitropropane.

Actually, where the group R₁ contains a primary or secondary amino group, it is usually protected during the above reaction, with a benzyl group being commonly used to protect a secondary amine and a benzyloxycarbonyl group being used to protect a primary amine. In either case, the protecting group in the product is removed by conventional procedures, for example by hydrogenation with hydrogen and a palladium catalyst.

Various procedures can be used to convert those compounds wherein A is =O and whose preparation is described below, to other different bridged derivatives of the present invention by standard methods. Thus, the ketone group in the polycyclic system can be reduced to the corresponding alcohol using an alkali metal (sodium or potassium) borohydride in a lower alkanol such as methanol or ethanol.

The ketone group can also be reduced completely to a methylene group by a two step procedure. In the first step, the ketone is reacted with ethylene dithiol or trimethylene dithiol in the presence of a strong acid such as hydrochloric acid or BF₃ to give the corresponding dithioketal. The reaction is carried out in a suitable polar solvent such as nitromethane or acetic acid. The dithioketal is then reduced with hydrazine in the presence of Raney nickel in a lower alkanol solvent such as 2-propanol at elevated temperatures (60-100°C). Actually this same procedure can be used to reduce the original starting alcohol, hexahydro-8-hydroxy-2,6-methano-2H-quinolizin-3(4H)-one, to 8-hydroxy-2,6-methanooctahydro-2H-quinolizine which can itself be reacted with acid derivatives as described earlier to give the corresponding esters.

Compounds containing other B-groups (i.e. aminomethyl, methylene or methyl groups) can be obtained from products in which A is =O and B is =H₂ by a Mannich reaction using formaldehyde and a secondary amine such as dimethylamine, diethylamine, piperidine or pyrrolidine. This reaction gives the corresponding aminomethyl compound and, when B is dimethylaminomethyl, the amino moiety is eliminated on heating at 90-110°C in an inert solvent such as toluene to give the corresponding methylene compound (B is =CH₂). This exocyclic methylene compound can be isolated by standard methods and transformed into a methyl group by hydrogenation, for example, by using hydrogen and platinum oxide.

To obtain those compounds in which A is hydroxyimino (=N-OH), the ketone referred to above can be reacted with hydroxylamine hydrochloride by standard procedures.

The alcohol used as a reactant in the above procedure can be obtained from known alkyl (C₁₋₄) 3-cyclopentene-1-carboxylates by a multi-step procedure. Specifically, the double bond in the indicated cyclopentene is oxidized to a 1,2-diol using N-methylmorpholine N-oxide in the presence of osmium tetroxide catalyst. The diol is then cleaved to the corresponding dialdehyde using sodium metaperiodate. A Robinson-Schöpf cyclization of the dialdehyde with a lower alkyl glycine ester and acetone-dicarboxylic acid, preferably at pH4, gives a pseudopelletierine derivative of the following type: 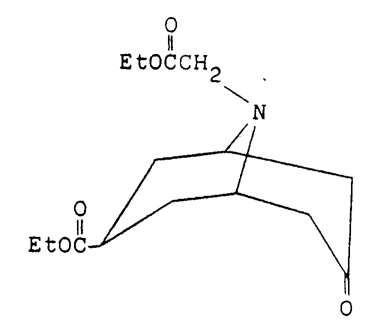
The ketone group is reduced to an alcohol using sodium borohydride and the product is reacted with dihydropyran to protect the -OH group as a tetrahydropyranyl ether. Dieckmann cyclization of the diester using a strong base (e.g. potassium t-butoxide) followed by aqueous acid hydrolysis and decarboxylation gives the desired alcohol. The resulting alcohols can exist in two conformations - axial and equatorial. The main product obtained by the above procedure is the axial alcohol and it can be separated from the equatorial isomer by crystallization of the camphorsulfonate or tetrafluoroborate salt.

The present compounds are useful for the treatment of pain, especially migraine vascular and cluster headaches and trigeminal neuralgia. They are also useful in the treatment of nausea and vomiting arising from treatment with cancer chemotherapeutic agents.

In the past, acute attacks of migraine have been treated with a peripheral vasoconstrictor, such as ergotamine, which may be co-administered with caffeine, and dihydroergotamine; an antipyretic analgesic, such as acetylsalicylic acid or p-acetylaminophenol; and/or an anti-emetic such as cyclizine, metoclopramide and thiethylperazine. It has also been reported (J. B. Hughes, Med. J. Aust. 2, No. 17, 580, 1977) that immediate relief of an acute migraine attack can be obtained by slow intravenous injection of metoclopramide (10 mg).

It is believed that 5-hydroxytryptamine (5-HT) is the naturally occurring substance most likely to play a role in the pathophysiology of migraine. Increased amounts of 5-HT and its metabolite 5-hydroxyindoleacetic acid are excreted in the urine during most attacks. Further, plasma and platelet 5-HT concentrations fall rapidly at the onset of an attack and remain low while the headache persists. Moreover, attacks of migraine have been clearly associated with periods of thrombocytopaenia in certain patients. It has been proposed that compounds which block the activity of 5-HT would be of use in the symptomatic treatment of migraine (J. R. Fozard, International Headache Congress 1980, reported in Advances in Neurology, Vol. 33, Raven Press, New York, 1982).

The known migraine prophylactic drugs, methysergide, propranolol, amitriptyline, and chlorpromazine have widely different pharmacological activities but all are 5-HT D-receptor antagonists at the doses used clinically for the prophylaxis of migraine. Metoclopramide is a potent 5-HT M-receptor antagonist and it has been proposed (J. R. Fozard supra) that a blockade of the M-receptor present on afferent sensory neurones affords symptomatic relief in an acute migraine attack.

The potency a 5-HT M-receptor antagonists of (-) cocaine and some related compounds, including pseudotropyl benzoate (i.e. benzoylpseudotropine) and 3,5-dichlorobenzoyltropine has been reported (J. R. Fozard et al., Eur. J. Pharmacol., 59, 1979, 195-210; J.R. Fozard, Naunyn-Schmiedeberg's Arch. Pharmacol., 326, 1984, 36-44). The pA₂ values reported for metoclopramide, pseudotropyl benzoate, nor(-) cocaine and benzoyltropine are 7.2, 7.0, 7.7 and 7.2 respectively whilst the pA₂ value determined for 3,5-dichlorobenzoyltropine by the same procedure is 9.3 (J. R. Fozard et al., Eur. J. Pharmacol., 49, 1978, 109-112; J.R. Fozard, Naunyn-Schmiedeberg's Arch. Pharmacol., 326, 1984, 36-44). In a double-blind clinical trial, 3,5-dichlorobenzoyltropine proved an effective treatment for the acute migraine attack (C. Loisy et al., Cephalalgia, 5, 1985, 79-82). A further series of tropine esters, with pA₂ values for blockade of the 5-HT M-receptors between 7.7 and 13.6 have been described by Richardson et al., Nature, 316, 1985, 126-131.
International Patent Applns. Publication No. WO 84/00166 and WO 85/01048 and European Patent Application Publication No. 189002 disclose mono or dicyclic or heterocyclic carbonic acid esters of piperidinol derivatives containing an alkylene bridge having a serotonin-M antagonistic action useful, in particular, as antimigraine and antiarhythmic agents.

The compounds of the present invention block the M-receptors for 5-hydroxytryptamine (5-HT) on afferent sensory neurones, certain of which subserve the transmission of pain. As explained above, the blocking of such M-receptors appears to be a mechanism whereby the symptoms of migraine can be relieved. Accordingly, the present compounds are useful in the treatment of migraine when administered in amounts sufficient to effectively block the said M-receptors.

In addition, compounds blocking 5-HT M-receptors, including metoclopramide, 3,5-dichlorobenzoyltropine and (3α-tropanyl)-1H-indole-3-carboxylic acid ester, are highly effective in preventing the nausea and vomiting induced by cancer chemotherapeutic agents in an animal experimental model (W.D. Miner et al., Brit. J. Pharmacol., 88, 1986, 374P; W.D. Miner and G.J. Sanger, Brit. J. Pharmacol., 88, 1986, 497-499; B. Costall et al., Neuropharmacology, 25, 1986, 959-961). It is believed that cytotoxic drug-induced vomiting involves a 5-HT M-receptor mechanism (W.D. Miner and G.J. Sanger, Brit. J. Pharmacol., 88, 1986, 497-499). Accordingly, the present compounds are useful in the treatment of cytotoxic drug-induced vomiting when administered in amounts sufficient to effectively block the said M-receptors. The activity of the compounds against 5-HT can be assessed by determining their pA₂ values in the isolated rabbit heart as described by J.R. Fozard et al., Eur. J. Pharmacol., 59, 195-210 (1979). In the method described, the molar concentration of antagonist which reduces the effects of twice the ED₅₀ of 5-HT to that of the ED₅₀ in the absence of antagonist is determined. The pA₂ value is the negative logarithm of said molar concentrations. In general terms, the higher the pA₂ value the more potent is the compound. When tested in this way, the present compounds show pA₂'s generally in the range of about 8 to 10.

The activity of these compounds against 5-HT can be assessed in vivo by measurement of the effect of the compound on the Von Bezold-Jarisch Reflex induced by 5-HT injected intravenously into the rat (see Paintal A.S., Physiol. Rev. 53, 159-227, 1973; J.R. Fozard, Naunyn-Schmiedeberg's Arch. Pharmacol., 326, 1984, 36-44). The transient cardiac slowing arises from an increased afferent vagus activity arising from stimulation by 5-HT of sensory afferent fibres in and around the heart. When tested against the Von Bezold-Jarisch Reflex induced by 5-HT, compounds endo-8-(3,5-dimethylbenzoyloxy)hexahydro-2,6-methano-2H-quinolizin-3(4H)-one hydrochloride and endo-hexahydro-8-(3-indolylcarbonyloxy)-2,6-methano-2H-quinolizin-3(4H)-one hydrochloride suppressed the response dose-dependently at doses of 0.01-0.1 mg/kg given intravenously or 0.25-1 mg/kg given orally.

The present compounds appear to be highly selective in their action against 5-HT M-receptor. Their potency against other 5-HT receptors and other spasmogens, in particular carbachol, phenylephrine, histamine and calcium, is known to be at least three orders lower than that against 5-HT M-receptors. Accordingly, their use in the treatment of migraine or cytotoxic drug-induced vomiting should be without any side effects.

The present compounds can be administered in various manners to achieve the desired effect. The compounds can be administered alone or in the form of pharmaceutical preparations to the patient being treated either orally or parenterally, for example, subcutaneously or intravenously. They can also be administered by inhalation or by suppository. The amount of compound administered will vary and can be any effective migraine-relieving amount or amount effective in cytotoxic drug vomiting. Depending upon the patient and the mode of administration, the quantity of compound administered may vary over a wide range to provide from about 0.01 mg/kg to about 10 mg/kg, usually 0.03 to 3.0 mg/kg, of body weight of the patient per dose. Unit doses of these compounds can contain, for example, from about 0.5 mg to 100 mg, usually 1 to 50 mg and preferably 3 to 30 mg, of the compound and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

Specific formulations of the present invention are prepared in a manner well known per se in the pharmaceutical art and usually comprise at least one active compound of the invention in admixture or otherwise in association with a pharmaceutically acceptable carrier or diluent therefor. For making those formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known per se. See Remington's Pharmaceutical Sciences, Mack Publishing Company, Easton, Pennsylvania, for a description of the preparation of such formulations.

The formulations of the invention may be adapted for enteral or parenteral use and may be administered to the patient in the form of tablets, capsules, suppositories, solutions, suspensions or the like.

The compounds of the present invention can be used in migraine therapy in combination with other antimigraine drugs having different modes of action. Such drugs include those used prophylactically, such as barbiturates, diazepam, chlorpromazine, amitriptyline, propranolol, methysergide, pizotifen, cyproheptadine, dihydroergotamine, and clonidine, and those used in the acute attack, such as vasoconstrictor agents, e.g., ergotamine and dihydroergotamine, analgesic/anti-inflammatory agents, e.g., aspirin, paracetamol and indomethacin, or anti-nauseants, e.g., cyclizine, metoclopramide, and thiethylperazine (see Fozard, J.R., J. Pharm. Pharmacol., 27, 297-321 (1975); Saper, J.R., J. Amer. Med. Assoc. 239, 480-484 (1978); Fozard,J.R., supra). As an example, compounds of the present invention would be beneficial in combination with aspirin 300-1200 mg or methysergide 2-6 mg given daily.

The following examples are presented to illustrate the present invention.

### EXAMPLE I

To a stirred solution of 160 g of diethyl malonate in 1.5 l of dry dimethylformamide at 0°C under nitrogen was slowly added 30 g of lithium hydride. After the evolution of hydrogen ceased (2 hours) 143 g of cis-1,4-dichloro-2-butene wad slowly added and the mixture allowed to come to room temperature. After 72 hours, the mixture was diluted with a mixture of ether and hexane (1:4) and poured into water. The organic layer was washed with water and brine before drying over magnesium sulfate. Distillation gave diethyl 3-cyclopentene-1,1-dicarboxylate, bp 70-80°C/0.1 mm Hg (13.332 Pa) containing a small amount (∼10%) of diethyl 2-vinylcyclopropane-1,1-dicarboxylate.

The impure cyclopentene diester (148.5 g) obtained above was added to a solution of 118 g of potassium hydroxide in 1333 ml of 80% ethanol and the stirred solution warmed at 60-70°C overnight. The ethanol was evaporated and the residue treated with an ice cold solution of concentrated sulphuric acid (107 ml) in water (274 ml). Extraction of the acid mixture with ether (3 x 400 ml) followed by evaporation of the dried ether extracts gave a residue of the diacid which was decarboxylated to the monoacid by heating in an oil bath at 170-180°C for 1 hour. The residual oil was distilled to give crude 3-cyclopentene-1-carboxylic acid, bp 68-73°C/1 mm Hg (133,32 Pa) containing some γ-vinyl-γ-butyrolactone. A solution of 98 g of potassium carbonate in 300 ml of water was added and the mixture extracted with ether to remove the γ-vinyl-γ-butyrolactone. Acidification of the aqueous solution and extraction with ether afforded pure 3-cyclopentene-1-carboxylic acid.

### EXAMPLE II

A mixture of 52 g of 3-cyclopentene-1-carboxylic acid and excess thionyl chloride was stirred at room temperature for 1 hour. The excess thionyl chloride evaporated and the residue distilled to give 3-cyclopentene-1-carbonyl chloride, bp 52-58°C.

The acid chloride obtained above was slowly added to an ice cooled stirred solution of 32 g of pyridine in 150 ml of ethanol. The mixture was stirred for a further hour, the ethanol evaporated and the residue treated with water and ether. The ether layer was separated, washed several times with water and dried. Evaporation of the ether left a residue of ethyl 3-cyclopentene-1-carboxylate, bp 62.5-66°C/14 mm Hg (1966,5 Pa).

### EXAMPLE III

A solution containing 84.6 g of N-methylmorpholine N-oxide, 1 g of osmium tetroxide, 230 ml of water and 115 ml of acetone was allowed to stir for 30 minutes at room temperature. To this stirred mixture was added very slowly over at least 8 hours, a solution of 80 g of ethyl 3-cyclopentene-1-carboxylate in 115 ml of acetone. The stirred mixture was heated at 50°C for 2 hours to complete the reaction (verified by TLC examination using ethyl acetate/hexane 70/30). Sodium bisulfite (∼10 g) was added, the stirring continued for a further 15 minutes, and the mixture filtered through Celite®. The pH of the filtrate was adjusted to 7 by the addition of 12 N sulfuric acid (37 ml), the acetone evaporated, the pH of the residual solution adjusted to 2 with 12 N sulfuric acid (13 ml) and the solution extracted with ethyl acetate (4 x 250 ml). Evaporation of the dried ethyl acetate solution gave 4-ethoxycarbonyl-1,2-cyclopentanediol.

### EXAMPLE IV

A solution of 85.4 g of sodium periodate in 500 ml of water was slowly added to a stirred solution of 69 g of 4-ethoxycarbonyl-1,2-cyclopentanediol in 690 ml of tetrahydrofuran. The reaction was exothermic and cooling was necessary. After two hours a precipitate of sodium iodate was filtered off and the solution concentrated at room temperature to remove most of the tetrahydrofuran. The resulting aqueous solution contained the desired β-ethoxycarbonylglutaraldehyde and was used directly in the next reaction.

To a stirred suspension of 400 g of potassium hydrogen phthalate in 800 ml of water was added, in sequence, a solution of 80 g of acetonedicarboxylic acid in 1200 ml of water, a solution of 80 g of glycine ethyl ester hydrochloride in 400 ml of water, and finally the solution of β-ethoxycarbonylglutaraldehyde obtained above. The mixture was stirred for 20 hours at room temperature during which time carbon dioxide evolved. The mixture was basified by the addition of an excess of aqueous potassium carbonate and extracted with ethyl acetate several times. Evaporation of the dried ethyl acetate extracts gave a syrup consisting mainly of 7-ethoxycarbonyl-9-(ethoxycarbonylmethyl)-9-azabicyclo-[3.3.1]nonan-3-one.

### EXAMPLE V

Sodium borohydride (17 g) was added in small portions to a stirred solution of 87.6 g of 7-ethoxycarbonyl-9-(ethoxycarbonylmethyl)-9-azabicyclo[3.3.1]nonan-3-one in 750 ml of ethanol. The mixture was stirred overnight at room temperature, the ethanol evaporated and the residue treated with 200 ml of water. Hydrochloric acid (2 M) was added until the mixture was acid and this acid solution was immediately basified by the addition of saturated potassium carbonate solution. Extraction with ethyl acetate and evaporation of the dried extract gave a syrup which consisted mainly of 7-ethoxycarbonyl-9-(ethoxycarbonylmethyl)-9-azabicyclo[3.3.1]nonan-3-ol. The syrup can be purified by column chromatography using silica and elution with hexane-ethyl acetate (30:70).

### EXAMPLE VI

A solution of 26.1 g of the crude 7-ethoxycarbonyl-9-(ethoxycarbonylmethyl)-9-azabicyclo[3.3.1]nonan-3-ol in 250 ml of methylene chloride was treated with one equivalent of methane-sulfonic acid (8.42 g). The methylene chloride solution was concentrated to about 35 ml, 9.5 ml of dihydropyran was added together with one drop of methanesulfonic acid, and the mixture stirred for 3 hours at room temperature. The mixture was then poured into saturated potassium carbonate solution and the product separated by extraction with ethyl acetate.

Evaporation of the dried ethyl acetate extracts gave a syrup consisting mainly of the tetrahydropyranyl ether of 7-ethoxycarbonyl-9-(ethoxycarbonylmethyl)-9-azabicyclo[3.3.1]nonan-3-ol. It can be purified by column chromatography using silica and elution with hexane-ethyl acetate (20:80), Rf 0.7.

### EXAMPLE VII

A solution of 34 g of the tetrahydropyranyl ether of 7-ethoxycarbonyl-9-(ethoxycarbonylmethyl)-9-azabicyclo|3.3.1|nonan-3-ol in 800 ml of anhydrous toluene was treated with 19 g of potassium tert-butoxide and the stirred mixture heated at 100°C for 2 hours. Anhydrous formic acid (7.85 g) was added to the cooled mixture, the potassium formate was filtered off, and the toluene solution evaporated to give a syrup. The syrup was treated with 300 ml of 5 N hydrochloric acid and the stirred solution refluxed overnight. The cooled mixture was clarified by an extraction with methylene chloride and the aqueous acid solution evaporated to dryness. The residue was dissolved in a little water and the solution treated with a large excess of saturated potassium carbonate solution. Extraction of the resulting mixture with ethyl acetate and evaporation of the dried ethyl acetate solution gave endo-hexahydro-8-hydroxy-2,6-methano-2H-quinolizin-3(4H)-one as an oil which crystallized on standing. The base was converted to its camphorsulfonate salt, m.p. 178°C, using one equivalent of camphorsulfonic acid in ethanol.

### EXAMPLE VIII

A mixture of 1.8 g of endo-hexahydro-8-hydroxy-2,6-methano-2H-quinolizin-3(4H)-one, hydrofluoroboric acid (0.88 g; 60% aqueous solution) and 20 ml of ethanol was evaporated, the residue was treated with 50 ml of anhydrous toluene, and the mixture again evaporated. A stirred suspension of the anhydrous residue in 50 ml of anhydrous nitroethane at -78°C was treated with 1.94 g of anhydrous silver tetrafluoroborate and a solution of 1.7 g of 3,5-dimethylbenzoyl chloride in 20 ml of anhydrous nitroethane was added slowly. The temperature of the stirred reaction was kept at -78°C for 1.5 hours and then allowed to return to room temperature overnight. Triethylamine (1 g) was added, the solution filtered and the nitroethane evaporated. A solution of the residue in 20 ml of water was treated with an excess of a saturated aqueous solution of potassium carbonate and the liberated oil separated by extraction with ethyl acetate. The ethyl acetate solution was washed several times with water before being dried over magnesium sulfate and evaporated. The residue obtained was endo-8-(3,5-dimethylbenzoyloxy)hexahydro-2,6-methano-2H-quinolizin-3(4H)-one and this was treated with methylene chloride and ethereal hydrogen chloride to give crystals of the hydrochloride salt melting at about 291°C.

### EXAMPLE IX

When the procedure of Example VIII is repeated using endo-hexahydro-8-hydroxy-2,6-methano-2H-quinolizin-3(4H)-one and the appropriate acid chloride, the corresponding esters listed below are obtained. As necessary, the acid chlorides were obtained from the appropriate carboxylic acids by standard procedures, for example, using thionyl chloride. To convert the ester to a corresponding acid salt, it was reacted with the appropriate acid with alternative solvents being used as desired.
endo-Hexahydro-8-(3-indolylcarbonyloxy)-2,6-methano-2H-quinolizin-3(4H)-one methanesulfonate melting at about 278°C.
endo-8-(3-Benzofurancarbonyloxy)hexanydro-2,6-methano-2H-quinolizin-3(4H)-one
endo-8-(3-Benzo[b]thiophenecarbonyloxy)hexahydro-2,6-methano-2H-quinolizin-3(4H)-one
endo-8-(1-Benzyl-1H-indol-3-ylcarbonyloxy)hexahydro-2,6-methano-2H-quinolizin-3(4H)-one
endo-Hexanydro-8-(1-methyl-1H-indol-3ylcarbonyloxy)-2,6-methano-2H-quinolizin-3(4H)-one
endo-8-(4-Bromo-2-furylcarbonyloxy)hexahydro-2,6-methano-2H-quinolizin-3(4H)-one
endo-Hexahydro-8-(5-phenyl-2-furylcarbonyloxy)-2,6-methano-2H-quinolizin-3(4H)-one
endo-8-(3-Chloro-2-thienylcarbonyloxy)hexahydro-2,6-methano-2H-quinolizin-3(4H)-one
endo-Hexahydro-8-(5-methyl-2-thienylcarbonyloxy)-2,6-methano-2H-quinolizin-3(4H)-one
endo-Hexahydro-8-(1-methyl-1H-pyrrol-2-ylcarbonyloxy)-2,6-methano-2H-quinolizin-3(4H)-one
endo-8-(3-Chloro-4-nitrobenzoyloxy)hexanydro-2,6-methano-2H-quinolizin-3(4H)-one
endo-8-(3-Chloro-4-dimethylaminobenzoyloxy)hexahydro-2,6-methano-2H-quinolizin-3(4H)-one
endo-8-(3,5-Dichlorobenzoyloxy)hexahydro-2,6-methano-2H-quinolizin-3(4H)-one
endo-8-(3,5-Dimethoxybenzoyloxy)hexahydro-2,6-methano-2H-quinolizin-3(4H)-one
endo-8-(3,5-Dimethylbenzoyloxy)hexahydro-2,6-methano-2H-quinolizin-3(4H)-one

### EXAMPLE X

Oxalyl chloride (0.76 ml) was slowly added to a stirred solution of 1 g of 5-methylindole in 20 ml of anhydrous ether at 0°C. The precipitate which formed was filtered off and dried at 80°C to give 5-methyl-3-indolylglyoxylyl chloride.

A stirred solution of 205 mg of anhydrous silver tetrafluoroborate in 10 ml of anhydrous nitroethane was treated with a solution of 282.5 mg of endo-hexahydro-8-hydroxy-2,6-methano-2H-quinolizin-3(4H)-one tetrafluoroborate (obtained by treating the free amine with an equivalent of hydrofluoroboric acid) in 10 ml of anhydrous nitroethane at room temperature. A solution of 233 mg of 5-methyl-3-indolylglyoxylyl chloride in 10 ml of anhydrous nitroethane was slowly added and the mixture stirred at room temperature overnight. Triethylamine (101 mg) was added, the solution filtered and the nitroethane evaporated. A solution of the residue in 15 ml of water was treated with a saturated aqueous solution of potassium carbonate and the liberated oil separated by extraction with ethyl acetate. The ethyl acetate solution was washed several times with water before being dried over magnesium sulfate and evaporated. The residue was treated with methylene chloride and ethereal hydrogen chloride, and the solid filtered off and recrystallized from 2-propanol to give endo-hexahydro-8-(5-methyl-3-indolylcarbonyloxy)-2,6-methano-2H-quinolizin-3(4H)-one hydrochloride.

When the above procedure was repeated using the appropriate substituted indole in place of the 5-methylindole, the following compounds were obtained:
endo-Hexahydro-8-(5-chloro-3-indolylcarbonyloxy)-2,6-methano-2H-quinolizin-3(4H)-one hydrochloride melting at about 317-320°C (with decomposition) after recrystallization from ethanol.
endo-Hexahydro-8-(5-cyano-3-indolylcarbonyloxy)-2,6-methano-2H-quinolizin-3(4H)-one hydrochloride melting at about 304-305°C, (with decomposition) after recrystallization from ethanol.
endo-Hexahydro-8-(5-methoxy-3-indolylcarbonyloxy)-2,6-methano-2H-quinolizin-3(4H)-one hydrochloride melting at about 303°C (with decomposition) after recrystallization from isopropanol.

Also obtained in the same way are endo-Hexahydro-8-(5-carbamoyl-3-indolylcarbonyloxy)-2,6-methano-2H-quinolizin-3(4H)-one and endo-Hexahydro-8-(5-hydroxy-3-indolycarbonyloxy)-2,6-methano-2H-quinolizin-3(4H)-one. In the latter case, the starting material is 5-benzyloxyindole and the initial product is debenzylated by reduction using standard procedures.

### EXAMPLE XI

Dimethylamine (40% solution in water, 0.68 g) and formaldehyde (30% solution in water, 0.49 g) were successively added to a solution of 1.25 g of endo-8-(3,5-dimethylbenzoyloxy)hexahydro-2,6-methano-2H-quinolizin-3(4H)-one in a mixture of 4 ml of ethanol and 2 ml of water. The stirred mixture was heated at 70-75°C for 16 hours and concentrated. Toluene (50 ml) was added and the mixture evaporated at 110°C.

A solution of the residue [which contained endo-8-(3,5-dimethylbenzoyloxy)hexahydro-4-methylene-2,6-methano-2H-quinolizin -3(4H)-one] in 30 ml of ethanol was hydrogenated at room temperature and atmospheric pressure in the presence of 0.2 g of platinum oxide (Adams catalyst). One equivalent of hydrogen was absorbed in one hour. The catalyst was filtered off, the ethanol evaporated and the residue treated with one equivalent of hydrofluoroboric acid in water. Evaporation of the aqueous solution gave a crystalline residue which was recrystallized from ethanol to give endo-8-(3,5-dimethylbenzoyloxy)hexahydro-4-methyl-2,6-methano-2H-quinolizin-3(4H)-one tetrafluoroborate melting at about 270-275°C.

### EXAMPLE XII

A solution of endo-8-(3-indolylcarbonyloxy)hexahydro-2,6-methano-2H-quinolizin-3(4H)-one (1.42 g) in ethanol (5 ml) was treated with fluoborio acid (0.64 g, 60% aqueous solution) and the mixture evaporated to give endo-8-(3-indolylcarbonyloxy)hexahydro-2,6-methano-2H-quinolizin-3(4H)-one tetrafluoroborate (1.8 g).

A stirred suspension of the above salt (1.8 g) in anhydrous nitroethane (30 ml) was treated with propane-1,3-dithiol (3 ml) and boron trifluoride etherate (3 drops) and the mixture stirred overnight at room temperature. The nitroethane was removed by evaporation and the residue triturated with ether. The solid product was filtered off, washed several times with ether, treated with water (25 ml), saturated aqueous potassium carbonate (3 ml) and ether (50 ml). The ether solution was separated off, dried (MgSO₄) and evaporated to give the propane dithioketal derivative, m.p. 226-229°C (1.6 g).

Hydrazine hydrate (3 ml) was added dropwise during one hour to a stirred refluxing solution of the above dithioketal (0.5 g) in isopropanol (20 ml) in the presence of Raney nickel (6 g, previously washed three times with isopropanol). The reflux was maintained for a further 30 minutes, the hot solution filtered through a triple superphosphate, the nickel washed several times with hot isopropanol and the combined filtrates evaporated to give endo-8-(3-indolylcarbonyloxy)-2,6-methanooctahydro-2H-quinolizine as the free base (50 mg). Addition of methylene chloride and ethereal hydrogen chloride gave the hydrochloride (30 mg), m.p. 311-313°C (from ethanol).

### EXAMPLE XIII

The procedure of Example XII was repeated using endo-hexahydro-8-hydroxy-2,6-methano-2H-quinolizin-3(4H)-one in place of the ester. The dithioketal obtained was reduced as described in the final paragraph except that the hydrazine hydrate was left out. This gave exo-octahydro-2,6-methano-2H-quinolizin-8-ol which was then reacted with 3,5-dimethylbenzoyl chloride to give exo-8-(3,5-dimethylbenzoyloxy)octahydro-2,6-methano-2H-quinolizine which was converted to the hydrochloride, m.p. 255-256°C by standard procedures.

## Claims (Claims for the following Contracting State(s): AT, BE, CH, DE, FR, GB, IT, LI, LU, NL, SE)

1. A compound of the formula 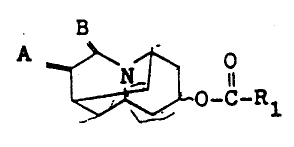 wherein A is =H₂, =O, =(H)(OH) or =N-OH; B is =H₂, =(H)(CH₃), =(H)(CH₂NR₃R₄) or =CH₂ wherein R₃ and R₄ arc C₂₋₄ alkyl or are combined to give tetramethylene, pentamethylene or -CH₂CH₂-O-CH₂CH₂-; R₁ is 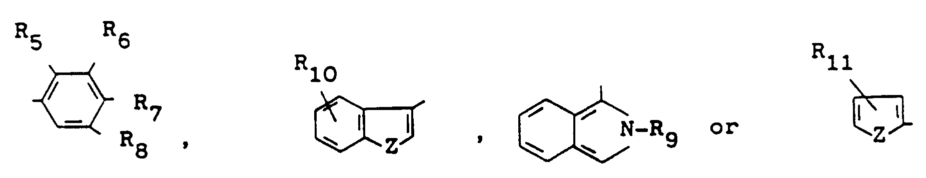 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; the wavy line indicates that the configuration of the oxygen substituent on the ring can be endo or exo; and the pharmaceutically acceptable acid addition and quaternary ammonium salts of the aforesaid compounds.

2. A compound according to Claim 1 which has the formula 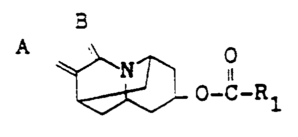 wherein A is =H₂, =O, =(H)(OH) or =N-OH; B is =H₂, =(H)(CH₃), =(H)(CH₂NR₃R₄) or =CH₂ wherein R₃ and R₄ are C₂₋₄ alkyl or are combined to give tetramethylene, pentamethylene or -CH₂CH₂-O-CH₂CH₂-; R₁ is 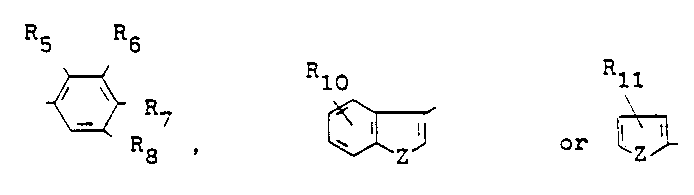 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl, and the pharmaceutically acceptable acid addition and quaternary ammonium salts of the aforesaid compounds.

3. A compound according to Claim 1 which has the formula 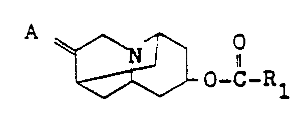 wherein A is =H₂, =O, =(H)(OH) or =N-OH; R₁ is 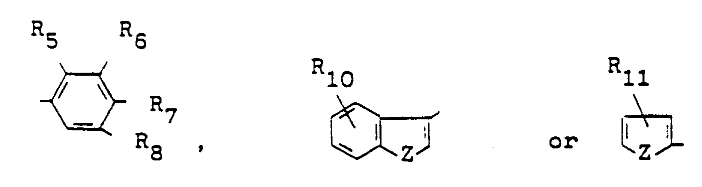 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl, and the pharmaceutically acceptable acid addition and quaternary ammonium salts of the aforesaid compounds.

4. A compound according to Claim 1 which has the formula 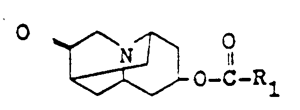 wherein R₁ is 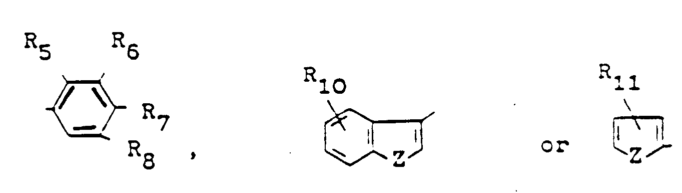 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; and the pharmaceutically acceptable acid addition and quaternary ammonium salts of the aforesaid compounds.

5. A compound according to Claim 1 which has the formula: 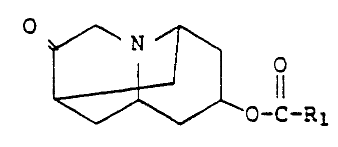 wherein R₁ is 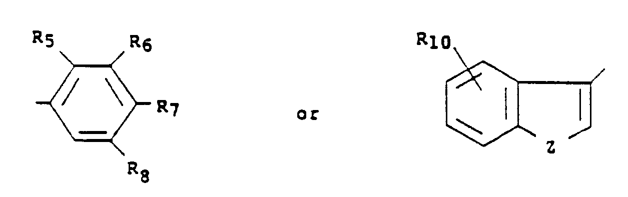 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂, and the pharmaceutically acceptable acid addition and quaternary ammonium salts of the aforesaid compounds.

6. A compound according to Claim 1 which is endo-8-(3,5-dimethylbenzoyloxy)hexahydro-2,6-methano-2H-quinolizin-3(4H)-one.

7. A compound according to Claim 1 which is endo-8-(3-indolylcarbonyloxy)hexahydro-2,6-methano-2H-quinolizin-3(4H)-one.

8. A compound according to claim 1, namely endo-Hexahydro-8-hydroxy-2,6-methano-2H-quinolizin-3(4H)-one.

9. A process for preparing a compound of claim 1 which comprises reacting an alcohol or a reactive derivative thereof, said alcohol having the formula: 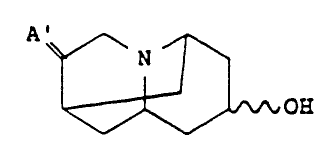 wherein A' is =H₂ or =O, with a reactive equivalent of an acid of the formula:
R₁COOH
wherein R₁ is as defined in claim 1, to give those compounds wherein A is =H₂ or =O, optionally followed by
(a) reduction of the product ketone with an alkali metal borohydride to give those compounds in which A is =(H)(OH), or
(b) conversion of the product ketone to a dithioketal with ethylene dithiol or trimethylene dithiol followed by reduction with hydrazine in the presence of Raney nickel to give those compounds in which A is =H₂, or
(c) reaction of the product ketone with hydroxylamine hydrochloride to give those compounds in which A is =N-OH, or
(d) reaction of the product ketone with formaldehyde and an appropriate secondary amine to give those compounds in which B is =(H)(CH₂NR₃R₄), followed by, when B is dimethylaminomethyl, heating to give those compounds in which B is =CH₂, further followed by hydrogenation to give those compounds in which B is =(H)(CH₃).

10. A process for preparing a compound of the formula 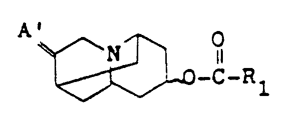 wherein A' is =H₂ or =O; R₁ is 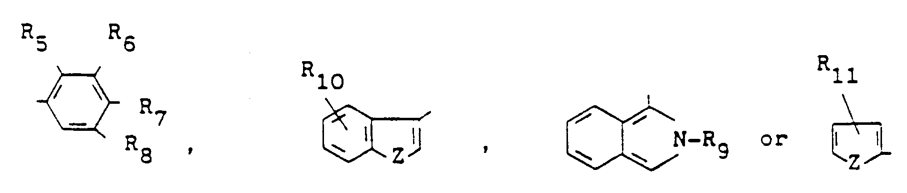 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; the wavy line indicates that the configuration of the oxygen substituent on the ring can be endo or exo; and the pharmaceutically acceptable acid addition and quaternary ammonium salts of the aforesaid compounds, which comprise reacting an alcohol or a reactive derivative thereof, said alcohol having the formula 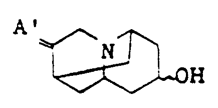 wherein A' is defined as above, with a reactive equivalent of an acid of the formula
R₁COOH
wherein R₁ is defined as above.

11. A process according to Claim 9 for preparing a compound of the formula 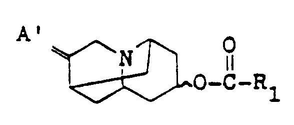 wherein A' is =H₂, or =O; R₁ is 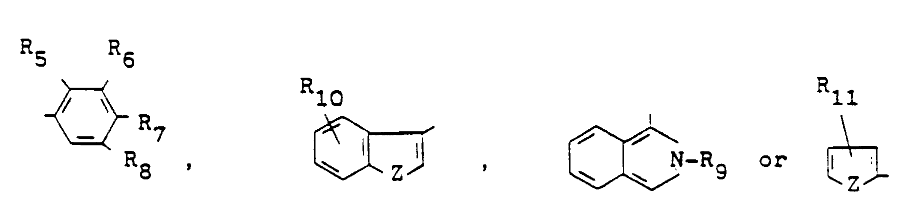 wherein R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; Z is NR₉, O or S; the configuration of the oxygen substituent on the ring as indicated by the wavy line is endo; and the pharmaceutically acceptable acid addition and quaternary ammonium salts of the aforesaid compounds, which comprise reacting an alcohol of the formula 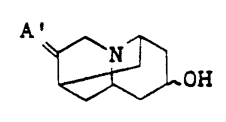 with an acid of the formula
R₁COOH
wherein A', R₁ and the wavy line are defined as above; said alcohol being used in the form of a super acid salt of the alcohol and in the presence of an equivalent of a heavy metal salt of the same super acid; said acid being used in the form of the corresponding acid chloride or bromide or the corresponding glyoxylyl chloride or bromide; with the reaction carried out in a nitroparaffin solvent at a temperature between -80°C and ambient temperature for a period up to about 24 hours.

12. A compound of claims 1, 2, 3, 4, 5, 6, 7 or 8 for use as a medicine.

13. Use of a compound of claims 1, 2, 3, 4, 5, 6, 7 or 8 for preparing a medicament for treating pain.

14. A pharmaceutical formulation containing a compound of claims 1, 2, 3, 4, 5, 6, 7 or 8 in admixture with a pharmaceutically acceptable carrier.

15. Use of a compound of claims 1, 2, 3, 4, 5, 6, 7 or 8 for preparing a medicament for treating cytotoxic drug induced vomiting.

16. Use of a compound of claims 1, 2, 3, 4, 5, 6, 7 or 8 for preparing a medicament for the therapy of migraine either alone or in combination with another antimigraine agent.

## Claims (Claims for the following Contracting State(s): ES)

1. A process for preparing a compound of the formula: 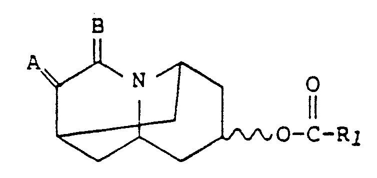 wherein A is =H₂, =O, =(H)(OH) or =N-OH; B is =H₂, =(H)(CH₃), =(H)(CH₂NR₃R₄) or =CH₂ wherein R₃ and R₄ are C₂₋₄ alkyl or are combined to give tetramethylene, pentamethylene or -CH₂CH₂-O-CH₂CH₂-; R₁ is 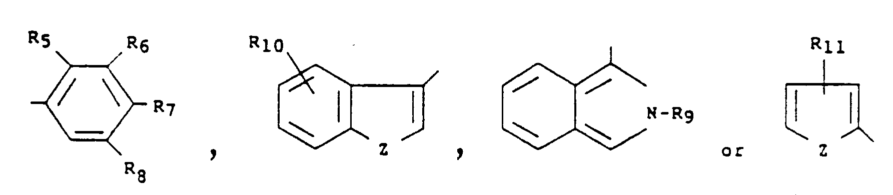 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; the wavy line indicates that the configuration of the oxygen substituent on the ring can be endo or exo; and the pharmaceutically acceptable acid addition and quaternary ammonium salts of the aforesaid compounds, which comprises reacting an alcohol or a reactive derivative thereof, said alcohol having the formula: 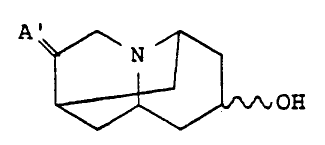 wherein A' is =H₂ or =O, with a reactive equivalent of an acid of the formula:
R₁COOH
wherein R₁ is defined as above, to give those compounds wherein A is =H₂ or =O, optionally followed by
(a) reduction of the product ketone with an alkali metal borohydride to give those compounds in which A is =(H)(OH), or
(b) conversion of the product ketone to a dithioketal with ethylene dithiol or trimethylene dithiol followed by reduction with hydrazine in the presence of Raney nickel to give those compounds in which A is =H₂, or
(c) reaction of the product ketone with hydroxylamine hydrochloride to give those compounds in which A is =N-OH, or
(d) reaction of the product ketone with formaldehyde and an appropriate secondary amine to give those compounds in which B is =(H)(CH₂NR₃R₄), followed by, when B is dimethylaminomethyl, heating to give those compounds in which B is =CH₂, further followed by hydrogenation to give those compounds in which B is =(H)(CH₃).

2. A process according to Claim 1 for preparing a compound of the formula: 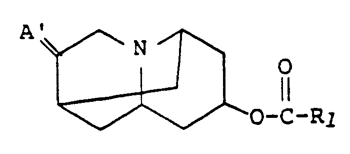 wherein A' is =H₂ or =O, R₁ is 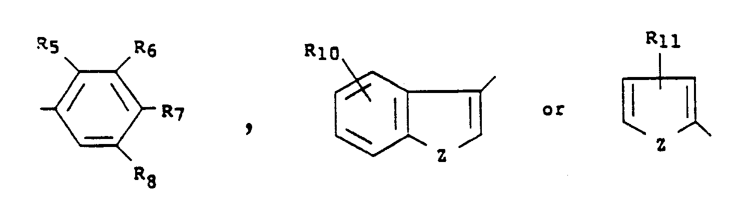 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; and the pharmaceutically acceptable acid addition and quaternary ammonium salts of the aforesaid compounds, which comprises reacting an alcohol or a reactive derivative thereof, said alcohol having the formula: 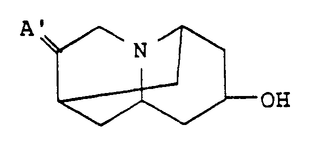 wherein A' is defined as above, with a reactive equivalent of an acid of the formula:
R₁COOH
wherein R₁ is defined as above.

3. A process according to Claim 1 for preparing a compound of the formula: 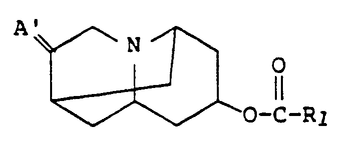 wherein A' is =H₂ or =O, R₁ is 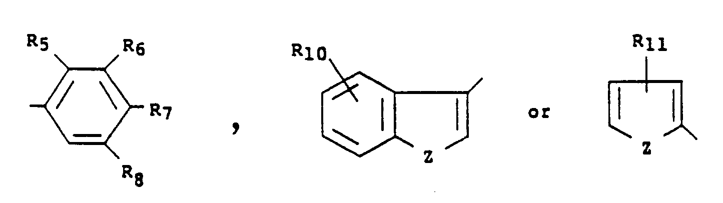 wherein R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; Z is NR₉, O or S; R₉ is hydrogen, C₁₋₄ alkyl or phenyl (C₁₋₂ alkyl); and the pharmaceutically acceptable acid addition and quaternary ammonium salts of the aforesaid compounds, which comprises reacting an alcohol of the formula: 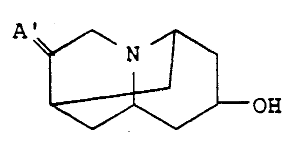 with an acid of the formula:
R₁COOH
wherein A' and R₁ are defined as above; said alcohol being used in the form of a super acid salt of the alcohol and in the presence of an equivalent of a heavy metal salt of the same super acid; said acid being used in the form of the corresponding acid chloride or bromide or the corresponding glyoxylyl chloride or bromide; with the reaction carried out in a nitroparaffin solvent at a temperature between -80°C and ambient temperature for a period up to about 24 hours.

4. A process according to Claim 1 for preparing a compound which has the formula: 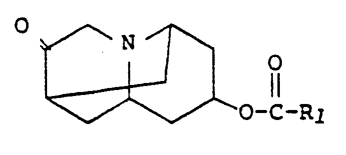 wherein R₁ is 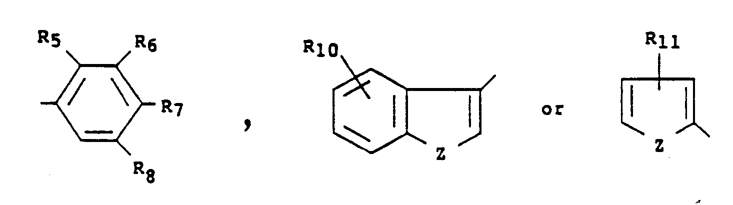 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; and the pharmaceutically acceptable acid addition and quaternary ammonium salts of the aforesaid compounds, which comprises reacting an alcohol of the formula: 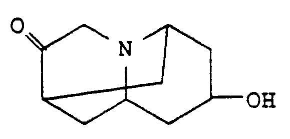 with an acid of the formula:
R₁COOH
wherein R₁ is defined as above; said alcohol being used in the form of a super acid salt of the alcohol and in the presence of an equivalent of a heavy metal salt of the same super acid; said acid being used in the form of the corresponding acid chloride or bromide or the corresponding glyoxylyl chloride or bromide; with the reaction carried out in a nitroparaffin solvent at a temperature between -80°C and ambient temperature for a period up to about 24 hours.

5. A process according to Claim 1 for preparing a compound which has the formula: 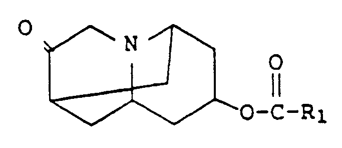 wherein R₁ is 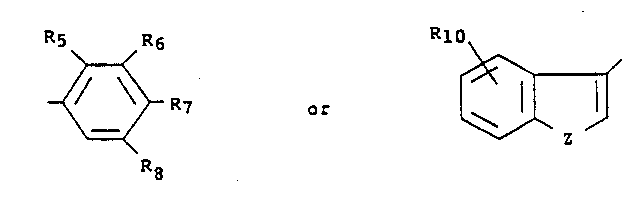 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂, and the pharmaceutically acceptable acid addition and quaternary ammonium salts of the aforesaid compounds, which comprises reacting an alcohol of the formula: 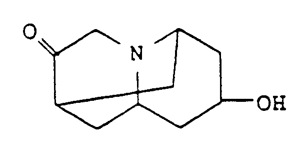 with an acid of the formula:
R₁COOH
wherein R₁ is defined as above; said alcohol being used in the form of a super acid salt of the alcohol and in the presence of an equivalent of a heavy metal salt of the same super acid; said acid being used in the form of the corresponding acid chloride or bromide or the corresponding glyoxylyl chloride or bromide; with the reaction carried out in a nitroparaffin solvent at a temperature between -80°C and ambient temperature for a period up to about 24 hours.

6. A process according to Claim 1 for preparing endo-8-(3,5-dimethylbenzoyloxy)hexahydro-2,6-methano-2H-quinolizin-3(4H)-one which comprises reacting endo-hexahydro-8-hydroxy-2,6-methano-2H-quinolizin-3(4H)-one first with hydrofluoroboric acid, then with silver tetrafluoroborate, and finally with 3,5-dimethylbenzoyl chloride.

7. A process according to Claim 1 for preparing endo-8-(3-indolylcarbonyloxy)hexahydro-2,6-methano-2H-quinolizin-3(4H)-one which comprises reacting endo-hexahydro-8-hydroxy-2,6-methano-2H-quinolizin-3(4H)-one first with hydrofluoroboric acid, then with silver tetrafluoroborate, and finally with indole-3-carboxylic acid chloride.

8. A process according to claim 1 for preparing end-hexahydro-8-hydroxy-2,6-methano-2H-quinolizin-3(4H)one.

9. A process for the preparation of a pharmaceutical composition comprising combining a compound as prepared according to any one of claims 1 to 8 with a pharmaceutically acceptable carrier.

10. A process according to claim 9 wherein the pharmaceutical composition prepared is useful in treating pain.

11. A process according to claim 9 wherein the pharmaceutical composition prepared is useful in treating cytotoxic drug induced vomiting.

12. A process according to claim 9 wherein the pharmaceutical composition prepared is useful in the therapy of migraine either alone or in combination with another antimigraine agent.

## Claims (Claims for the following Contracting State(s): GR)

1. A compound of the formula 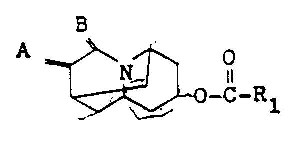 wherein A is =H₂, =O, =(H)(OH) or =N-OH; B is =H₂, =(H)(CH₃), =(H)(CH₂NR₃R₄) or =CH₂ wherein R₃ and R₄ are C₂₋₄ alkyl or are combined to give tetramethylene, pentamethylene or -CH₂CH₂-O-CH₂CH₂-; R₁ is 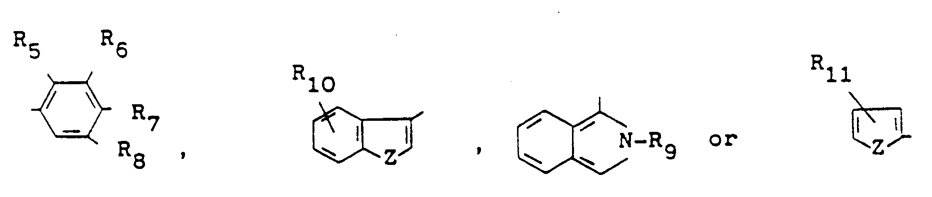 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; the wavy line indicates that the configuration of the oxygen substituent on the ring can be endo or exo; and the pharmaceutically acceptable acid addition and quaternary ammonium salts of the aforesaid compounds.

2. A compound according to Claim 1 which has the formula 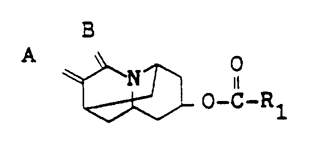 wherein A is =H₂, =O, =(H)(OH) or =N-OH; B is =H₂, =(H)(CH₃), =(H)(CH₂NR₃R₄) or =CH₂ wherein R₃ and R₄ are C₂₋₄ alkyl or are combined to give tetramethylene, pentamethylene or -CH₂CH₂-O-CH₂CH₂-; R₁ is 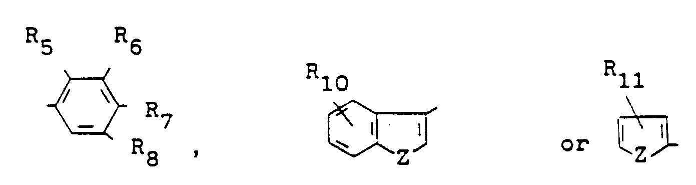 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl, and the pharmaceutically acceptable acid addition and quaternary ammonium salts of the aforesaid compounds.

3. A compound according to Claim 1 which has the formula 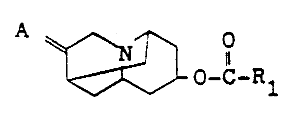 wherein A is =H₂, =O, =(H)(OH) or =N-OH; R₁ is 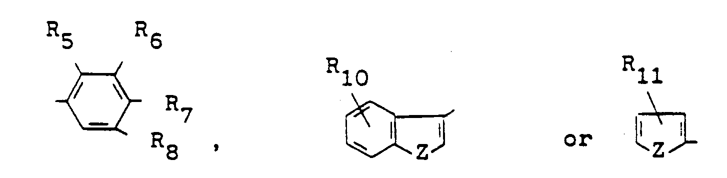 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl, and the pharmaceutically acceptable acid addition and quaternary ammonium salts of the aforesaid compounds.

4. A compound according to Claim 1 which has the formula 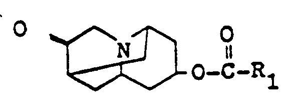 wherein R₁ is 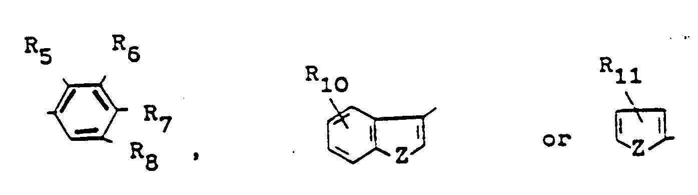 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; and the pharmaceutically acceptable acid addition and quaternary ammonium salts of the aforesaid compounds.

5. A compound according to Claim 1 which has the formula: 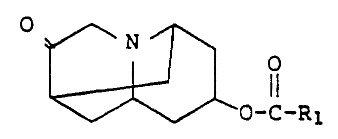 wherein R₁ is 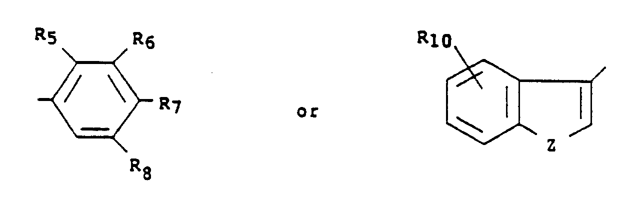 wherein Z is NR₉, O or S; R₅, R₆ and R₈ are each hydrogen, halogen, C₁₋₃ alkyl or C₁₋₃ alkoxy; R₇ is hydrogen, amino, C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂, and the pharmaceutically acceptable acid addition and quaternary ammonium salts of the aforesaid compounds.

6. A compound according to Claim 1 which is endo-8-(3,5-dimethylbenzoyloxy)hexahydro-2,6-methano-2H-quinolizin-3(4H)-one.

7. A compound according to Claim 1 which is endo-8-(3-indolylcarbonyloxy)hexahydro-2,6-methano-2H-quinolizin-3(4H)-one.

8. A compound according to claim 1, namely endo-Hexahydro-8-hydroxy-2,6-methano-2H-quinolizin-3(4H)-one.

9. A process for preparing a compound of claim 1 which comprises reacting an alcohol or a reactive derivative thereof, said alcohol having the formula: 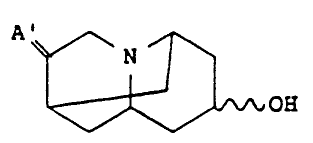 wherein A' is =H₂ or =O, with a reactive equivalent of an acid of the formula:
R₁COOH
wherein R₁ is as defined in claim 1, to give those compounds wherein A is =H₂ or =O, optionally followed by
(a) reduction of the product ketone with an alkali metal borohydride to give those compounds in which A is =(H)(OH), or
(b) conversion of the product ketone to a dithioketal with ethylene dithiol or trimethylene dithiol followed by reduction with hydrazine in the presence of Raney nickel to give those compounds in which A is =H₂, or
(c) reaction of the product ketone with hydroxylamine hydrochloride to give those compounds in which A is =N-OH, or
(d) reaction of the product ketone with formaldehyde and an appropriate secondary amine to give those compounds in which B is =(H)(CH₂NR₃R₄), followed by, when B is dimethylaninomethyl, heating to give those compounds in which B is =CH₂, further followed by hydrogenation to give those compounds in which B is =(H)(CH₃).

10. A process for preparing a compound of the formula 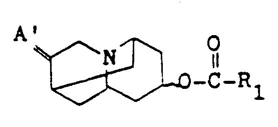 wherein A' is =H₂ or =O; R₁ is 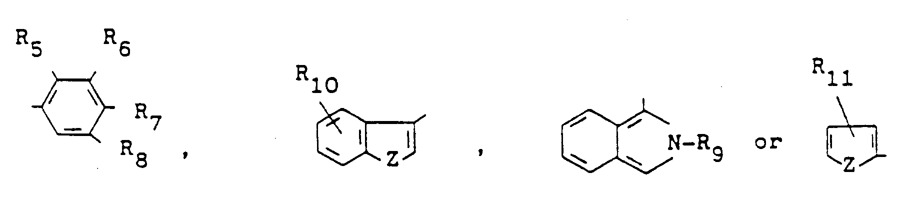 wherein Z is NR₉, O or S; R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; the wavy line indicates that the configuration of the oxygen substituent on the ring can be endo or exo; and the pharmaceutically acceptable acid addition and quaternary ammonium salts of the aforesaid compounds, which comprise reacting an alcohol or a reactive derivative thereof, said alcohol having the formula 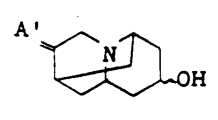 wherein A' is defined as above, with a reactive equivalent of an acid of the formula
R₁COOH
wherein R₁ is defined as above.

11. A process according to Claim 9 for preparing a compound of the formula 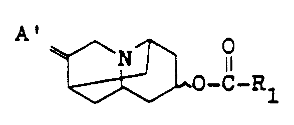 wherein A' is =H₂, or =O; R₁ is 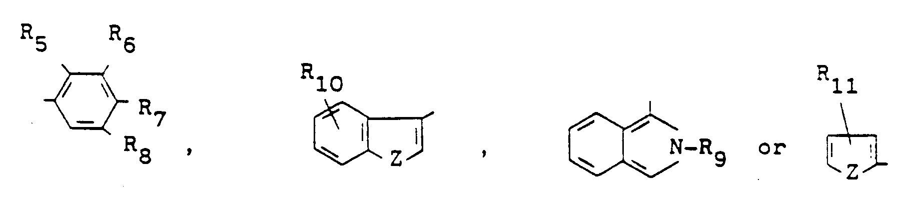 wherein R₅, R₆ and R₈ are each hydrogen, halogen, C₁₋₃ alkyl or C₁₋₃ alkoxy; R₇ is hydrogen, amino, (C₁₋₄ alkyl)amino, (C₁₋₄ alkyl)₂amino or nitro; R₉ is hydrogen, C₁₋₄ alkyl or phenyl (C₁₋₂ alkyl); R₁₀ is hydrogen, halogen, C₁₋₄ alkyl, C₁₋₄ alkoxy, hydroxy, cyano or -CONH₂; R₁₁ is hydrogen, halogen, C₁₋₄ alkyl or phenyl; Z is NR₉, O or S; the configuration of the oxygen substituent on the ring as indicated by the wavy line is endo; and the pharmaceutically acceptable acid addition and quaternary ammonium salts of the aforesaid compounds, which comprise reacting an alcohol of the formula 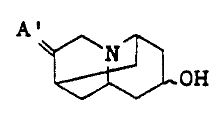 with an acid of the formula
R₁COOH
wherein A', R₁ and the wavy line are defined as above; said alcohol being used in the form of a super acid salt of the alcohol and in the presence of an equivalent of a heavy metal salt of the same super acid; said acid being used in the form of the corresponding acid chloride or bromide or the corresponding glyoxylyl chloride or bromide; with the reaction carried out in a nitroparaffin solvent at a temperature between -80°C and ambient temperature for a period up to about 24 hours.

12. A process for the preparation of a pharmaceutical composition comprising combining a compound as defined in any one of claims 1 to 8 with a pharmaceutically acceptable carrier.

13. A process according to claim 12 wherein the pharmaceutical composition is useful in treating pain.

14. A process according to claim 12 wherein the pharmaceutical composition is useful in treating cytotoxic drug induced vomiting.

15. A process according to claim 12 wherein the pharmaceutical composition is useful in the therapy of migraine either alone or in combination with another antimigraine agent.

## Patentansprüche (Patentansprüche für folgende(n) Vertragsstaat(en): AT, BE, CH, DE, FR, GB, IT, LI, LU, NL, SE)

1. Verbindung der Formel 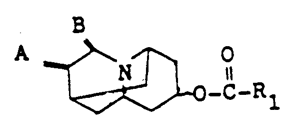 in der A =H₂, =O, =(H)(OH) oder =N-OH darstellt; B = H₂, =(H)(CH₃), =(H)(CH₂NR₃R₄) oder =CH₂ darstellt, wobei R₃ und R₄ C₂₋₄-Alkylreste bedeuten oder zusammengesetzt eine Tetramethylen-, Pentamethylengruppe oder eine Gruppe -CH₂CH₂-O-CH₂CH₂- ergeben; R₁ einen Rest 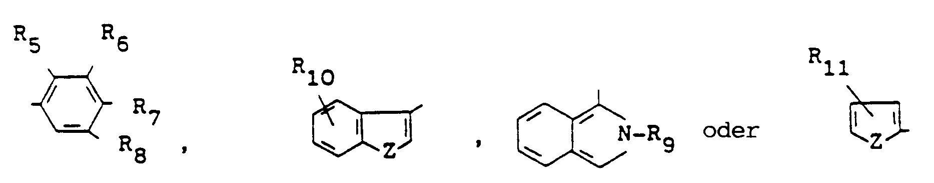 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; in der die geschlängelte Linie anzeigt, daß die Konfiguration des Sauerstoffsubstituenten am Ring endo oder exo sein kann; sowie die pharmazeutisch verträglichen Säureadditionssalze und quatären Ammoniumsalze der vorstehend erwähnten Verbindungen.

2. Verbindung nach Anspruch 1 mit der Formel 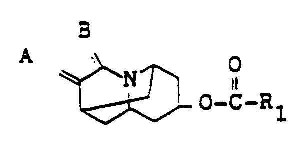 in der A =H₂, =O, =(H)(OH) oder =N-OH darstellt; B =H₂, =(H)(CH₃), =(H)(CH₂NR₃R₄) oder =CH₂ darstellt, wobei R₃ und R₄ C₂₋₄-Alkylreste bedeuten oder zusanmengesetzt eine Tetramethylen-, Pentamethylengruppe oder eine Gruppe -CH₂CH₂-O-CH₂CH₂- ergeben; R₁ einen Rest 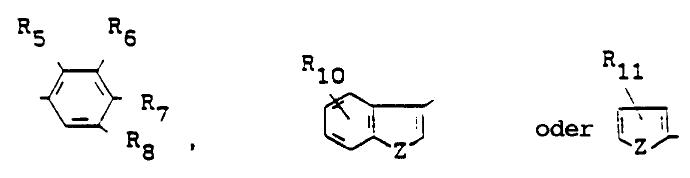 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt, sowie die pharmazeutisch verträglichen Säureadditionssalze und quatären Ammoniumsalze der vorstehend erwähnten Verbindungen.

3. Verbindung nach Anspruch 1 mit der Formel 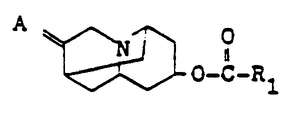 in der A = H₂, =O, =(H)(OH) oder =N-OH darstellt; R₁ einen Rest 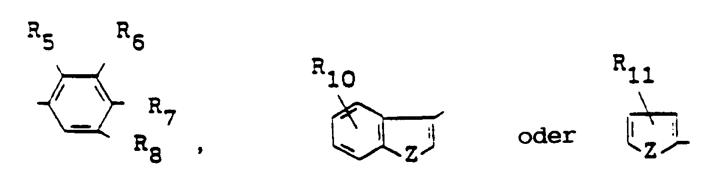 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt, sowie die pharmazeutisch verträglichen Säureadditionssalze und quatären Ammoniumsalze der vorstehend erwähnten Verbindungen.

4. Verbindung nach Anspruch 1 mit der Formel 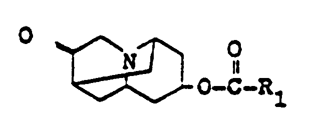 in der R₁ einen Rest 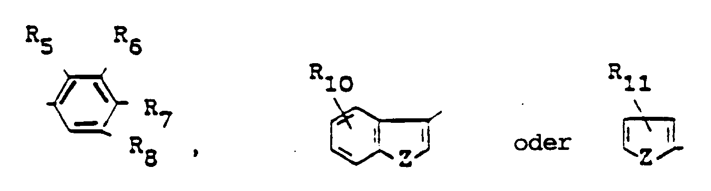 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt, sowie die pharmazeutisch verträglichen Säureadditionssalze und quatären Ammoniumsalze der vorstehend erwähnten Verbindungen.

5. Verbindung nach Anspruch 1 mit der Formel 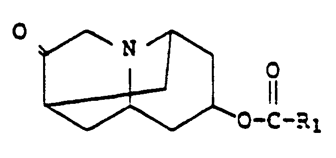 in der R₁ einen Rest 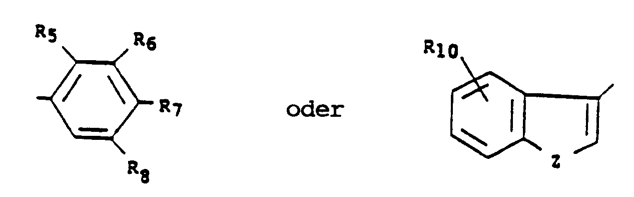 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl-oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy, Cyanorest oder -CONH₂-Rest darstellt, sowie die pharmazeutisch verträglichen Säureadditionssalze und quatären Ammoniumsalze der vorstehend erwähnten Verbindungen.

6. Verbindung nach Anspruch 1, bei der es sich um endo-8-(3,5-Dimethylbenzoyloxy)hexahydro-2,6-methano-2H-chinolizin-3(4H)-on handelt.

7. Verbindung nach Anspruch 1, bei der es sich um endo-8-(3-Indolylcarbonyloxy)hexahydro-2,6-methano-2H-chinolizin-3(4H)-on handelt.

8. Verbindung nach Anspruch 1, bei der es sich um endo-Hexahydro-8-hydroxy-2,6-methano-2H-chinolizin-3(4H)-on handelt.

9. Verfahren zur Herstellung einer Verbindung nach Anspruch 1, umfassend die Umsetzung eines Alkohols oder eines reaktiven Derivats davon, wobei der Alkohol die Formel: 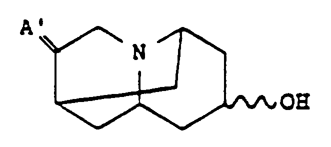 hat, in der A' =H₂ oder =O darstellt, mit einem reaktiven Äquivalent einer Säure der Formel:
R₁COOH
in der R₁ wie in Anspruch 1 definiert ist, wobei sich diejenigen Verbindungen ergeben, in denen A =H₂ oder =O darstellt, gegebenenfalls gefolgt von
(a) Reduktion des Produktketons mit einem Alkalimetall-borhydrid, wobei sich diejenigen Verbindungen ergeben, in denen A =(H)(OH) bedeutet, oder
(b) Überführen des Produktketons mit Ethylendithiol oder Trimethylendithiol in ein Dithioketal, gefolgt von von der Reduktion mit Hydrazin in der Gegenwart von Raney-Nickel, wobei sich diejenigen Verbindungen ergeben, in denen A =H₂ bedeutet, oder
(c) Umsetzung des Produktketons mit Hydroxylamin-Hydrochlorid, wobei sich diejenigen Verbindungen ergeben, in den A =N-OH bedeutet, oder
(d) Umsetzung des Produktketons mit Formaldehyd und einem geeigneten sekundären Amin, wobei sich diejenigen Verbindungen ergeben, in denen B =(H)(CH₂NR₃R₄) bedeutet, gefolgt von Erwärmen, falls B eine Dimethylaminomethylgruppe bedeutet, wobei sich diejenigen Verbindungen ergeben, in denen B =CH₂ bedeutet, im weiteren gefolgt von Hydrierung, wobei sich diejenigen Verbindungen ergeben, in denen B =(H)(CH₃) bedeutet.

10. Verfahren zur Herstellung einer Verbindung der Formel 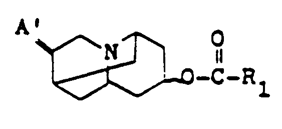 in der A' =H₂ oder =O darstellt; R₁ einen Rest 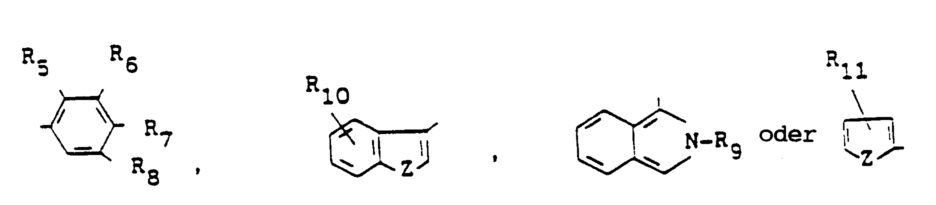 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; in der die geschlängelte Linie anzeigt, daß die Konfiguration des Sauerstoffsubstituenten am Ring endo oder exo sein kann; sowie die pharmazeutisch verträglichen Säureadditionssalze und quatären Ammoniumsalze der vorstehenden Verbindungen, umfassend die Umsetzung eines Alkohols oder eines reaktiven Derivats davon, wobei der Alkohol die Formel 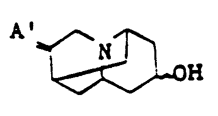 hat, in der A' wie vorstehend definiert ist, mit einem reaktiven Äquivalent einer Säure der Formel
R₁COOH
in der R₁ wie vorstehend definiert ist.

11. Verfahren nach Anspruch 9 zur Herstellung einer Verbindung der Formel 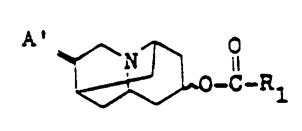 in der A' =H₂ oder =O darstellt, R₁ einen Rest 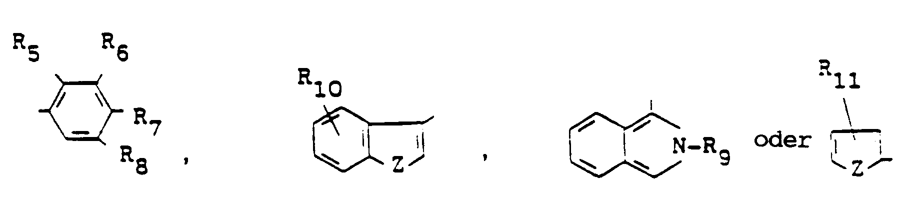 darstellt, wobei R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; Z NR₉, O oder S bedeutet; in der die Konfiguration des Sauerstoffsubstituenten am Ring, die durch die geschlängelte Linie angedeutet wird, endo ist; sowie die pharmazeutisch verträglichen Säureadditionssalze und quatären Ammoniumsalze der vorstehenden Verbindungen, umfassend die Umsetzung eines Alkohols der Formel 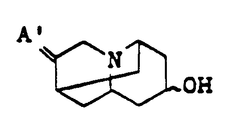 mit einer Säure der Formel
R₁COOH
wobei A', R₁ und die geschlängelte Linie wie vorstehend definiert sind; wobei der Alkohol in der Form eines Supersäuresalzes des Alkohols und in der Gegenwart eines Äquivalents eines Schwermetallsalzes der gleichen Supersäure verwendet wird; die Säure in der Form des entsprechenden Säurechlorids oder -bromids oder des entsprechenden Glyoxylylchlorids oder -bromids verwendet wird; und die Umsetzung in einem Nitroparaffin-Lösungsmittel bei einer Temperatur zwischen -80°C und Umgebungstemperatur innerhalb eines Zeitraums bis zu etwa 24 Stunden durchgeführt wird.

12. Verbindung nach Anspruch 1, 2, 3, 4, 5, 6, 7 oder 8 zur Verwendung als Arzneimittel.

13. Verwendung einer Verbindung nach Anspruch 1, 2, 3, 4, 5, 6, 7 oder 8 zur Herstellung eines Arzneimittels zur Schmerzbehandlung.

14. Pharmazeutische Formulierung, die eine Verbindung nach Anspruch 1, 2, 3, 4, 5, 6, 7 oder 8 in Beimischung mit einem pharmazeutisch verträglichen Träger enthält.

15. Verwendung einer Verbindung nach Anspruch 1, 2, 3, 4, 5, 6, 7 oder 8 zur Herstellung eines Arzneimittels zur Behandlung von Erbrechen, das durch cytotoxische Medikamente hervorgerufen wird.

16. Verwendung einer Verbindung nach Anspruch 1, 2, 3, 4, 5, 6, 7 oder 8 zur Herstellung eines Arzneimittels zur Migränetherapie, entweder alleine oder in Kombination mit einem anderen Antimigränemittel.

## Patentansprüche (Patentansprüche für folgende(n) Vertragsstaat(en): ES)

1. Verfahren zur Herstellung einer Verbindung der Formel 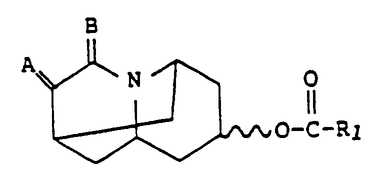 in der A =H₂, =O, =(H)(OH) oder =N-OH darstellt; B =H₂, =(H)(CH₃), =(H)(CH₂NR₃R₄) oder =CH₂ darstellt, wobei R₃ und R₄ C₂₋₄-Alkylreste bedeuten oder zusammengesetzt eine Tetramethylen-, Pentamethylengruppe oder eine Gruppe -CH₂CH₂-O-CH₂CH₂- ergeben; R₁ einen Rest 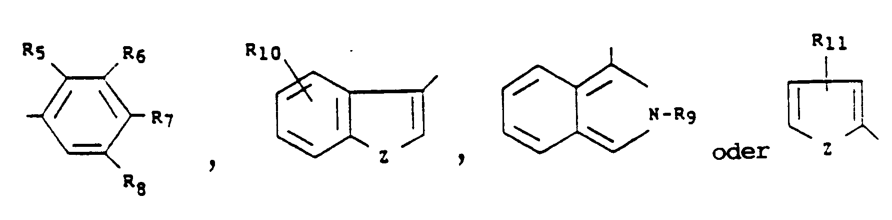 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; in der die geschlängelte Linie anzeigt, daß die Konfiguration des Sauerstoffsubstituenten am Ring endo oder exo sein kann; sowie der pharmazeutisch verträglichen Säureadditionssalze und quatären Ammoniumsalze der vorstehend erwähnten Verbindungen, umfassend die Umsetzung eines Alkohols oder eines reaktiven Derivats davon, wobei der Alkohol die Formel 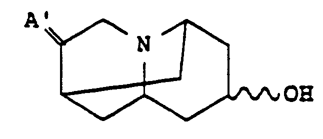 hat, in der A' =H₂ oder =O darstellt, mit einem reaktiven Äquivalent einer Säure der Formel
R₁COOH
in der R₁ wie vorstehend definiert ist, wobei sich diejenigen Verbindungen ergeben, in denen A =H₂ oder =O ist, gegebenenfalls gefolgt von
(a) Reduktion des Produktketons mit einem Alkalimetall-borhydrid, wobei sich diejenigen Verbindungen ergeben, in denen A =(H)(OH) bedeutet, oder
(b) Überführen des Produktketons mit Ethylendithiol oder Trimethylendithiol in ein Dithioketal, gefolgt von von der Reduktion mit Hydrazin in der Gegenwart von Raney-Nickel, wobei sich diejenigen Verbindungen ergeben, in denen A =H₂ bedeutet, oder
(c) Umsetzung des Produktketons mit Hydroxylamin-Hydrochlorid, wobei sich diejenigen Verbindungen ergeben, in den A =N-OH bedeutet, oder
(d) Umsetzung des Produktketons mit Formaldehyd und einem geeigneten sekundären Amin, wobei sich diejenigen Verbindungen ergeben, in denen B =(H)(CH₂NR₃R₄) bedeutet, gefolgt von Erwärmen, falls B eine Dimethylaminomethylgruppe bedeutet, wobei sich diejenigen Verbindungen ergeben, in denen B =CH₂ bedeutet, im weiteren gefolgt von Hydrierung, wobei sich diejenigen Verbindungen ergeben, in denen B =(H)(CH₃) bedeutet.

2. Verfahren nach Anspruch 1 zur Herstellung einer Verbindung der Formel 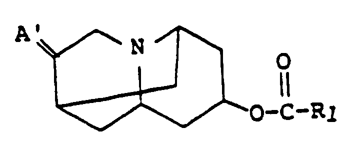 in der A' =H₂ oder =O darstellt, R₁ einen Rest 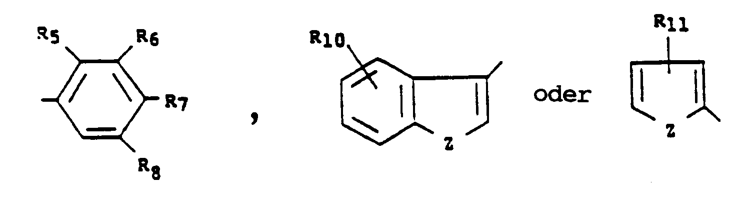 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; sowie der pharmazeutisch verträglichen Säureadditionssalze und quatären Ammoniumsalze der vorstehend erwähnten Verbindungen, umfassend die Umsetzung eines Alkohols oder eines reaktiven Derivats davon, wobei der Alkohol die Formel: 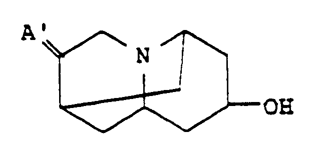 hat, in der A' wie vorstehend definiert ist, mit einem reaktiven Äquivalent einer Säure der Formel:
R₁COOH
in der R₁ wie vorstehend definiert ist.

3. Verfahren nach Anspruch 1 zur Herstellung einer Verbindung der Formel 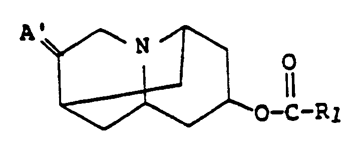 in der A' =H₂ oder =O darstellt, R₁ einen Rest 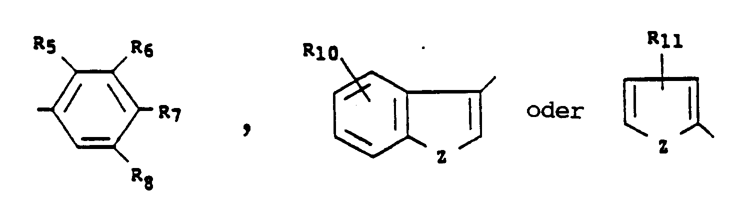 darstellt, wobei R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; Z NR₉, O oder S bedeutet; R₉ ein Wasserstoffatom, einen C₁₋₄-Alkyl- oder Phenyl-(C₁₋₂-alkyl)-rest darstellt; sowie der pharmazeutisch verträglichen Säureadditionssalze und quatären Ammoniumsalze der vorstehend erwähnten Verbindungen, umfassend die Umsetzung eines Alkohols der Formel: 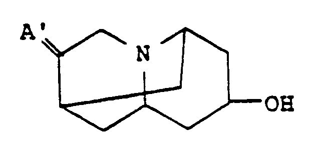 mit einer Säure der Formel:
R₁COOH
wobei A' und R₁ wie vorstehend definiert sind; wobei der Alkohol in der Form eines Supersäuresalzes des Alkohols und in der Gegenwart eines Äquivalents eines Schwermetallsalzes der gleichen Supersäure verwendet wird; die Säure in der Form des entsprechenden Säurechlorids oder -bromids oder des entsprechenden Glyoxylylchlorids oder -bromids verwendet wird; und die Umsetzung in einem Nitroparaffin-Lösungsmittel bei einer Temperatur zwischen -80°C und Umgebungstemperatur innerhalb eines Zeitraums bis zu etwa 24 Stunden durchgeführt wird.

4. Verfahren nach Anspruch 1 zur Herstellung einer Verbindung der Formel 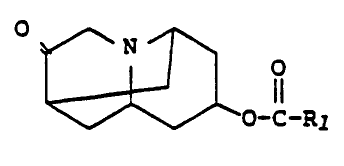 in der R₁ einen Rest 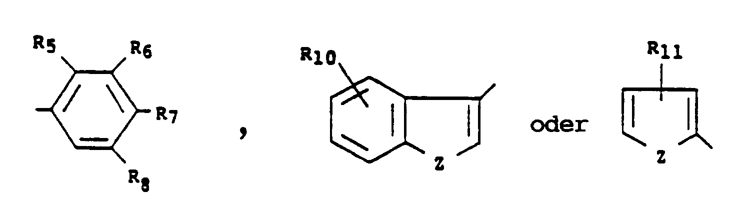 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; sowie der pharmazeutisch verträglichen Säureadditionssalze und quatären Ammoniumsalze der vorstehenden Verbindungen, umfassend die Umsetzung eines Alkohols der Formel 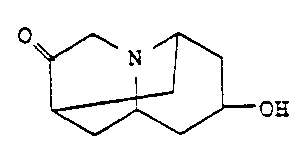 mit einer Säure der Formel
R₁COOH
in der R₁ wie vorstehend definiert ist; wobei der Alkohol in der Form eines Supersäuresalzes des Alkohols und in der Gegenwart eines Äquivalents eines Schwermetallsalzes der gleichen Supersäure verwendet wird; die Säure in der Form des entsprechenden Säurechlorids oder -bromids oder des entsprechenden Glyoxylylchlorids oder -bromids verwendet wird; und die Umsetzung in einem Nitroparaffin-Lösungsmittel bei einer Temperatur zwischen -80°C und Umgebungstemperatur innerhalb eines Zeitraums bis zu etwa 24 Stunden durchgeführt wird.

5. Verfahren nach Anspruch 1 zur Herstellung einer Verbindung der Formel 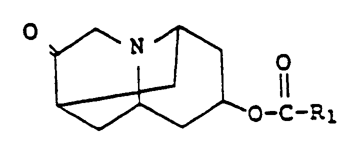 in der R₁ einen Rest 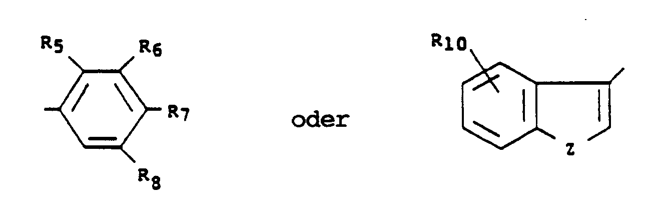 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; sowie der pharmazeutisch verträglichen Säureadditionssalze und quatären Ammoniumsalze der vorstehenden Verbindungen, umfassend die Umsetzung eines Alkohols der Formel 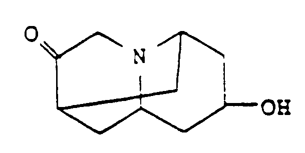 mit einer Säure der Formel
R₁COOH
in der R₁ wie vorstehend definiert ist; wobei der Alkohol in der Form eines Supersäuresalzes des Alkohols und in der Gegenwart eines Äquivalents eines Schwermetallsalzes der gleichen Supersäure verwendet wird; die Säure in der Form des entsprechenden Säurechlorids oder -bromids oder des entsprechenden Glyoxylylchlorids oder -bromids verwendet wird; und die Umsetzung in einem Nitroparaffin-Lösungsmittel bei einer Temperatur zwischen -80°C und Umgebungstemperatur innerhalb eines Zeitraums bis zu etwa 24 Stunden durchgeführt wird.

6. Verfahren nach Anspruch 1 zur Herstellung von endo-8-(3,5-Dimethylbenzoyloxy)hexahydro-2,6-methano-2H-chinolizin-3(4H)-on, umfassend die Umsetzung von endo-Hexahydro-8-hydroxy-2,6-methano-2H-chinolizin-3(4H)-on zuerst mit Hydrofluoroborsäure, dann mit Silbertetrafluoroborat und schließlich mit 3,5-Dimethylbenzoylchlorid.

7. Verfahren nach Anspruch 1 zur Herstellung von endo-8-(3-Indolylcarbonyloxy)hexahydro-2,6-methano-2H-chinolizin-3(4H)-on, umfassend die Umsetzung von endo-Hexahydro-8-hydroxy-2,6-methano-2H-chinolizin-3(4H)-on zuerst mit Hydrofluoroborsäure, dann mit Silbertetrafluoroborat und schließlich mit Indol-3-carbonsäurechlorid.

8. Verfahren nach Anspruch 1 zur Herstellung von endo-Hexahydro-8-hydroxy-2,6-methano-2H-chinolizin-3(4H)-on.

9. Verfahren zur Herstellung eines Arzneimittels, umfassend die Kombination einer Verbindung, wie sie nach irgendeinem der Ansprüche 1 bis 8 hergestellt wurde, mit einem pharmazeutisch verträglichen Träger.

10. Verfahren nach Anspruch 9, wobei das hergestellte Arzneimittel zur Schmerzbehandlung geeignet ist.

11. Verfahren nach Anspruch 9, wobei das hergestellte Arzneimittel zur Behandlung von Erbrechen, das durch cyctotoxische Medikamente hervorgerufen wird, geeignet ist.

12. Verfahren nach Anspruch 9, wobei das hergestellte Arzneimittel entweder alleine oder in Kombination mit einem anderen Antimigränemittel für die Migränetherapie geeignet ist.

## Patentansprüche (Patentansprüche für folgende(n) Vertragsstaat(en): GR)

1. Verbindung der Formel 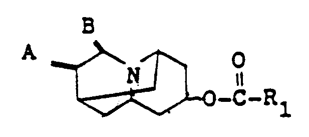 in der A =H₂, =O, =(H)(OH) oder =N-OH darstellt; B =H₂, =(H)(CH₃), =(H)(CH₂NR₃R₄) oder =CH₂ darstellt, wobei R₃ und R₄ C₂₋₄-Alkylreste bedeuten oder zusammengesetzt eine Tetramethylen-, Pentamethylengruppe oder eine Gruppe -CH₂CH₂-O-CH₂CH₂- ergeben; R₁ einen Rest 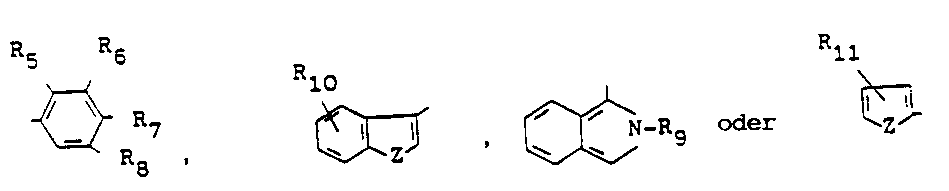 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; in der die geschlängelte Linie anzeigt, daß die Konfiguration des Sauerstoffsubstituenten am Ring endo oder exo sein kann; sowie die pharmazeutisch verträglichen Säureadditionssalze und quatären Ammoniumsalze der vorstehend erwähnten Verbindungen.

2. Verbindung nach Anspruch 1 mit der Formel 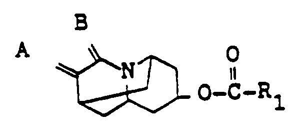 in der A =H₂, =O, =(H)(OH) oder =N-OH darstellt; B =H₂, =(H)(CH₃), =(H)(CH₂NR₃R₄) oder =CH₂ darstellt, wobei R₃ und R₄ C₂₋₄-Alkylreste bedeuten oder zusammengesetzt eine Tetramethylen-, Pentamethylengruppe oder eine Gruppe -CH₂CH₂-O-CH₂CH₂- ergeben; R₁ einen Rest 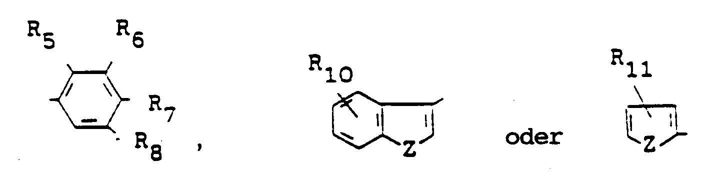 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt, sowie die pharmazeutisch verträglichen Säureadditionssalze und quatären Ammoniumsalze der vorstehend erwähnten Verbindungen.

3. Verbindung nach Anspruch 1 mit der Formel 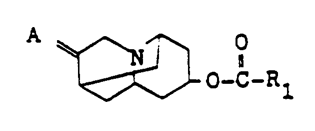 in der A =H₂, =O, =(H)(OH) oder =N-OH darstellt; R₁ einen Rest 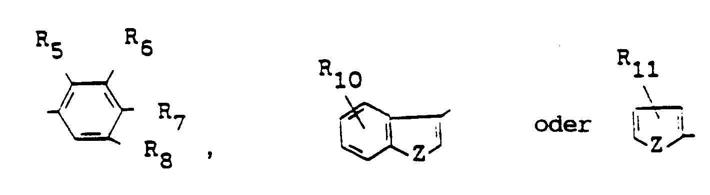 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt, sowie die pharmazeutisch verträglichen Säureadditionssalze und quatären Ammoniumsalze der vorstehend erwähnten Verbindungen.

4. Verbindung nach Anspruch 1 mit der Formel 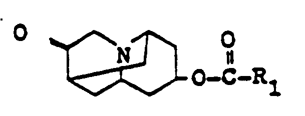 in der R₁ einen Rest 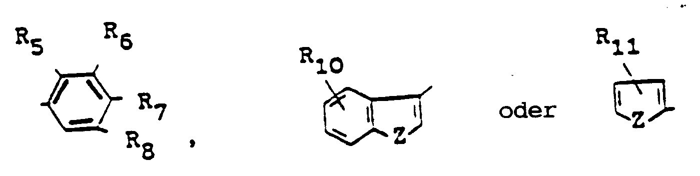 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt, sowie die pharmazeutisch verträglichen Säureadditionssalze und quatären Ammoniumsalze der vorstehend erwähnten Verbindungen.

5. Verbindung nach Anspruch 1 mit der Formel 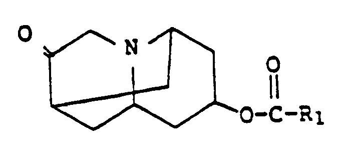 in der R₁ einen Rest 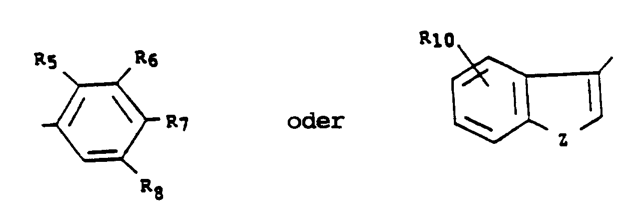 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl-oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt, sowie die pharmazeutisch verträglichen Säureadditionssalze und quatären Ammoniumsalze der vorstehend erwähnten Verbindungen.

6. Verbindung nach Anspruch 1, bei der es sich um endo-8-(3,5-Dimethylbenzoyloxy)hexahydro-2,6-methano-2H-chinolizin-3(4H)-on handelt.

7. Verbindung nach Anspruch 1, bei der es sich um endo-8-(3-Indolylcarbonyloxy)hexahydro-2,6-methano-2H-chinolizin-3(4H)-on handelt.

8. Verbindung nach Anspruch 1, bei der es sich um endo-Hexahydro-8-hydroxy-2,6-methano-2H-chinolizin-3(4H)-on handelt.

9. Verfahren zur Herstellung einer Verbindung nach Anspruch 1, umfassend die Umsetzung eines Alkohols oder eines reaktiven Derivats davon, wobei der Alkohol die Formel: 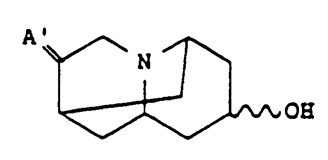 hat, in der A' =H₂ oder =O darstellt, mit einem reaktiven Äquivalent einer Säure der Formel:
R₁COOH
in der R₁ wie in Anspruch 1 definiert ist, wobei sich diejenigen Verbindungen ergeben, in denen A =H₂ oder =O darstellt, gegebenenfalls gefolgt von
(a) Reduktion des Produktketons mit einem Alkalimetall-borhydrid, wobei sich diejenigen Verbindungen ergeben, in denen A =(H)(OH) bedeutet, oder
(b) Überführen des Produktketons mit Ethylendithiol oder Trimethylendithiol in ein Dithioketal, gefolgt von von der Reduktion mit Hydrazin in der Gegenwart von Raney-Nickel, wobei sich diejenigen Verbindungen ergeben, in denen A =H₂ bedeutet, oder
(c) Umsetzung des Produktketons mit Hydroxylamin-Hydrochlorid, wobei sich diejenigen Verbindungen ergeben, in den A =N-OH bedeutet, oder
(d) Umsetzung des Produktketons mit Formaldehyd und einem geeigneten sekundären Amin, wobei sich diejenigen Verbindungen ergeben, in denen B =(H)(CH₂NR₃R₄) bedeutet, gefolgt von Erwärmen, falls B eine Dimethylaminomethylgruppe bedeutet, wobei sich diejenigen Verbindungen ergeben, in denen B =CH₂ bedeutet, im weiteren gefolgt von Hydrierung, wobei sich diejenigen Verbindungen ergeben, in denen B =(H)(CH₃) bedeutet.

10. Verfahren zur Herstellung einer Verbindung der Formel 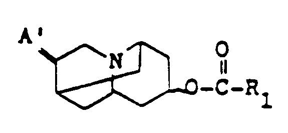 in der A' =H₂ oder =O darstellt; R₁ einen Rest 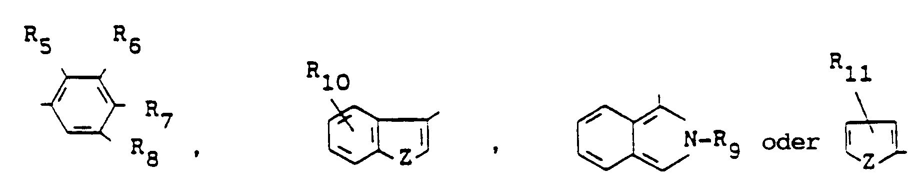 darstellt, wobei Z NR₉, O oder S bedeutet; R₅, R₆ und R₈ jeweils ein Wasserstoffatom, einen Halogen-, C₁₋₃-Alkyl- oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; in der die geschlängelte Linie anzeigt, daß die Konfiguration des Sauerstoffsubstituenten am Ring endo oder exo sein kann; sowie die pharmazeutisch verträglichen Säureadditionssalze und quatären Ammoniumsalze der vorstehenden Verbindungen, umfassend die Umsetzung eines Alkohols oder eines reaktiven Derivats davon, wobei der Alkohol die Formel 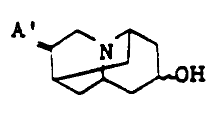 hat, in der A' wie vorstehend definiert ist, mit einem reaktiven Äquivalent einer Säure der Formel
R₁COOH
in der R₁ wie vorstehend definiert ist.

11. Verfahren nach Anspruch 9 zur Herstellung einer Verbindung der Formel 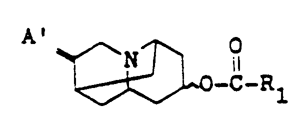 in der A' =H₂ oder =O darstellt, R₁ einen Rest 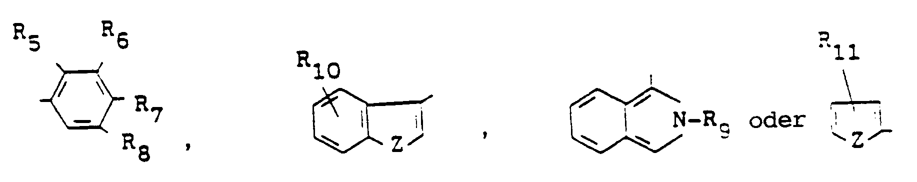 darstellt, wobei R₅, R₆ und R₈ jeweils ein Wasserstoffatom, einen Halogen-, C₁₋₃-Alkyl oder C₁₋₃-Alkoxyrest darstellen; R₇ ein Wasserstoffatom, einen Amino-, (C₁₋₄-Alkyl)amino-, (C₁₋₄-Alkyl)₂amino- oder Nitrorest darstellt, R₉ ein Wasserstoffatom, einen C₁₋₄-Alkyl- oder Phenyl-(C₁₋₂-alkyl)-rest darstellt; R₁₀ ein Wasserstoffatom, einen Halogen-, C₁₋₄-Alkyl-, C₁₋₄-Alkoxy-, Hydroxy-, Cyanorest oder -CONH₂-Rest darstellt; R₁₁ ein Wasserstoffatom, einen Halogen-, C₁₋₄-Alkyl- oder Phenylrest darstellt; Z NR₉, O oder S bedeutet; in der die Konfiguration des Sauerstoffsubstituenten am Ring, die durch die geschlängelte Linie angedeutet wird, endo ist; sowie die pharmazeutisch verträglichen Säureadditionssalze und quatären Ammoniumsalze der vorstehenden Verbindungen, umfassend die Umsetzung eines Alkohols der Formel 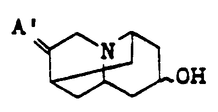 mit einer Säure der Formel
R₁COOH
wobei A', R₁ und die geschlängelte Linie wie vorstehend definiert sind; wobei der Alkohol in der Form eines Supersäuresalzes des Alkohols und in der Gegenwart eines Äquivalents eines Schwermetallsalzes der gleichen Supersäure verwendet wird; die Säure in der Form des entsprechenden Säurechlorids oder -bromids oder des entsprechenden Glyoxylylchlorids oder -bromids verwendet wird; und die Umsetzung in einem Nitroparaffin-Lösungsmittel bei einer Temperatur zwischen -80°C und Umgebungstemperatur innerhalb eines Zeitraums bis zu etwa 24 Stunden durchgeführt wird.

12. Verfahren zur Herstellung eines Arzneimittels, umfassend die Kombination einer Verbindung, wie sie nach irgendeinem der Ansprüche 1 bis 8 definiert wurde, mit einem pharmazeutisch verträglichen Träger.

13. Verfahren nach Anspruch 12, wobei das Arzneimittel zur Schmerzbehandlung geeignet ist.

14. Verfahren nach Anspruch 12, wobei das Arzneimittel zur Behandlung von Erbrechen, das durch cyctotoxische Medikamente hervorgerufen wird, geeignet ist.

15. Verfahren nach Anspruch 12, wobei das Arzneimittel entweder alleine oder in Kombination mit einem anderen Antimigränemittel für die Migränetherapie geeignet ist.

## Revendications (Revendications pour l'(les) Etat(s) contractant(s) suivant(s): AT, BE, CH, DE, FR, GB, IT, LI, LU, NL, SE)

1. Un composé de formule: 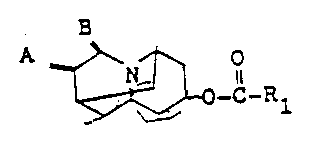 dans laquelle A est =H₂, =O, =(H)(OH) ou =N-OH; B est =H₂, =(H)(CH₃), =(H)(CH₂NR₃R₄) ou =CH₂, où R₃ et R₄ sont des groupes C₂-C₄ alkyles ou sont combinés pour donner un groupe tétraméthylène, pentaméthylène ou -CH₂CH₂-O-CH₂CH₂- ; R₁ est : 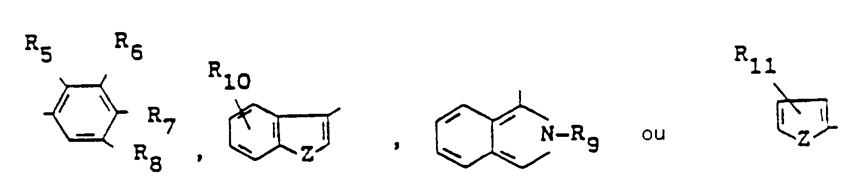 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; le trait ondulé indique que la configuration du substituant oxygène sur le noyau peut être endo ou exo ; et les sels d'addition d'acide et les sels d'ammonium quaternaire pharmaceutiquement acceptables desdits composés.

2. Un composé selon la revendication 1 qui a la formule : 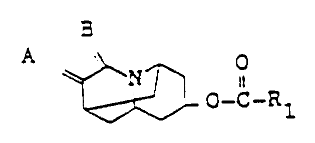 dans laquelle A est =H₂, =O, =(H)(OH) ou =N-OH ; B est =H₂, =(H)(CH₃), =(H)(CH₂NR₃R₄) ou =CH₂, dans laquelle R₃ et R₄ sont des groupes C₂-C₄ alkyles ou sont combinés pour donner un groupe tétraméthylène, pentaméthylène ou -CH₂CH₂-O-CH₂CH₂- ; R₁ est : 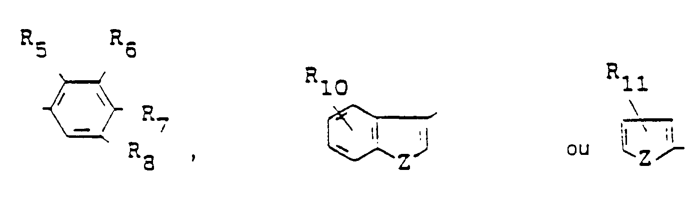 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle, et les sels d'addition d'acide et les sels d'ammonium quaternaire pharmaceutiquement acceptables desdits composés.

3. Un composé selon la revendication 1 qui a la formule : 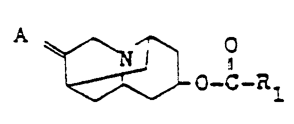 dans laquelle A est =H₂, =O, =(H)(OH) ou =N-OH; R₁ est : 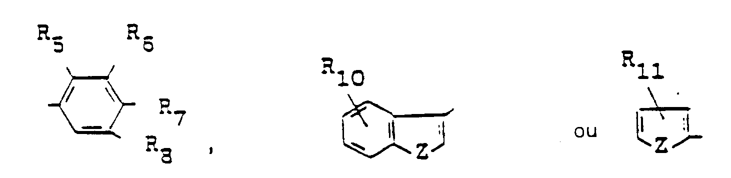 où Z est NR₉, O ou S; R₅, R₆ et R₈ désignent chacun un atome d'hydrogène, d'halogène, un groupe C₁-C₃ alkyle ou C₁-C₃ alcoxy; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle); R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle, et les sels d'addition d'acide ou les sels d'ammonium quaternaire pharmaceutiquement acceptables desdits composés.

4. Un composé selon la revendication 1 qui a la formule : 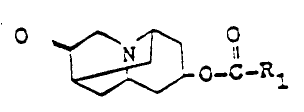 dans laquelle R₁ est : 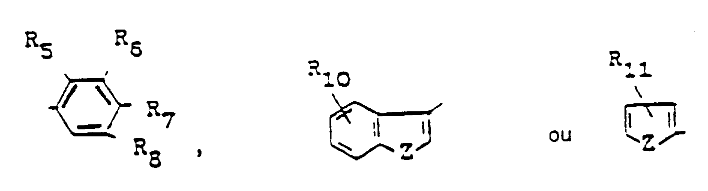 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle, et les sels d'addition d'acide et les sels d'ammonium quaternaire pharmaceutiquement acceptables desdits composés.

5. Un composé selon la revendication 1 qui a la formule : 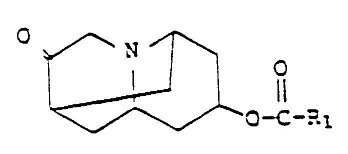 dans laquelle R₁ est : 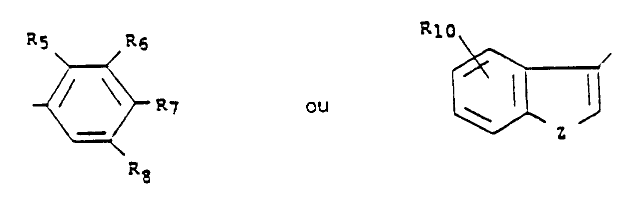 où Z est NR₉, O ou S ; R₅, R₆ et R₈ désignent chacun un atome d'hydrogène, d'halogène, un groupe C₁-C₃ alkyle ou C₁-C₃ alcoxy; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂, et les sels d'addition d'acide et les sels d'ammonium quaternaire pharmaceutiquement acceptables desdits composés.

6. Un composé selon la revendication 1 qui est l'endo-8-(3,5-diméthylbenzoyloxy)hexahydro-2,6-méthano-2H-quinolizine-3(4H)-one.

7. Un composé selon la revendication 1 qui est l'endo-8-(3-indolylcarbonyloxy)hexahydro-2,6-méthano-2H-quinolizine-3(4H)-one.

8. Un composé selon la revendication 1 qui est l'endo-hexahydro-8-hydroxy-2,6-méthano-2H-quinolizine-3(4H)-one.

9. Un procédé de préparation d'un composé selon la revendication 1 qui comprend la réaction d'un alcool ou d'un dérivé réactif de ce dernier, ledit alcool ayant la formule : 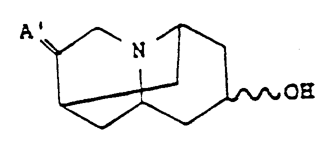 dans laquelle A' est =H₂ ou =O, avec un équivalent réactif d'un acide de formule :
R₁COOH
dans laquelle R₁ est comme défini dans la revendication 1, pour donner des composés dans lesquels A est =H₂ ou =O, éventuellement suivie de
(a) la réduction de la cétone produite avec a borohydrure de métal alcalin pour donner les composés dans lesquels A est =(H)(OH), ou bien
(b) la conversion de la cétone produite en un dithiocétal avec l'éthylènedithiol ou le triméthylènedithiol suivie de la réduction par l'hydrazine en présence du nickel Raney pour donner les composés dans lesquels A est =H₂, ou bien
(c) la réaction de la cétone produite avec le chlorhydrate d'hydroxylamine pour donner les composés dans lesquels A est =N-OH, ou bien
(d) la réaction de la cétone produite avec le formaldéhyde et une amine secondaire appropriée pour donner les composés dans lesquels B est =(H)(CH₂NR₃R₄), suivie, lorsque B est un groupe diméthylaminométhyle, d'un chauffage pour donner les composés dans lesquels B est =CH₂, puis suivie de l'hydrogénation pour donner les composés dans lesquels B est =(H)(CH₃).

10. Un procédé de préparation d'un composé de formule : 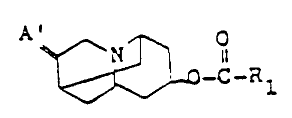 dans laquelle A' est =H₂, =O; R₁ est : 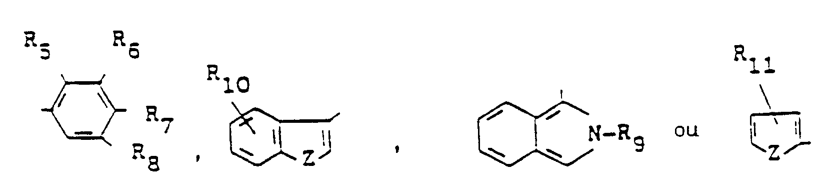 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; le trait ondulé indique que la configuration du substituant oxygène sur le noyau peut être endo ou exo ; et les sels d'addition d'acide et les sels d'ammonium quaternaire pharmaceutiquement acceptables desdits composés, qui comprend la réaction d'un alcool ou d'un dérivé réactif de ce dernier, ledit alcool ayant la formule : 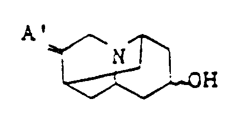 dans laquelle A' est défini comme précédemment, avec un équivalent réactif d'un acide de formule :
R₁COOH
dans laquelle R₁ est défini comme précédemment.

11. Un procédé selon la revendication 9 pour préparer un composé de formule : 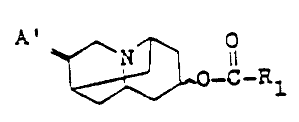 dans laquelle A' est =H₂ ou =O; R₁ est : 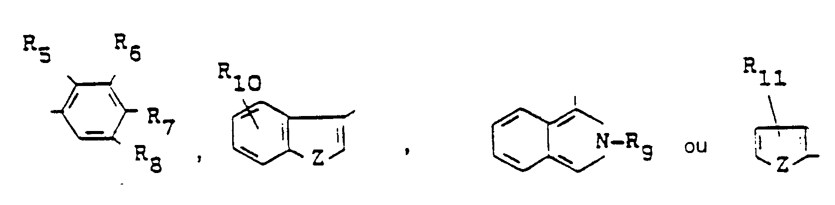 où R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; Z est NR₉, O ou S ; la configuration du substituant oxygène sur le noyau comme indiqué par le trait ondulé est endo ; et les sels d'addition d'acide et les sels d'ammonium quaternaire pharmaceutiquement acceptables desdits composés, qui comprend la réaction d'un alcool de formule : 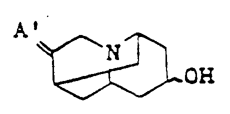 avec un acide de formule :
R₁COOH
dans laquelle A', R₁ et le trait ondulé sont définis comme précédemment ; ledit alcool étant utilisé sous la forme d'un sel de superacide de l'alcool et en présence d'un équivalent d'un sel de métal lourd du même superacide ; ledit acide étant utilisé sous la forme du chlorure ou du bromure d'acide correspondant ou du chlorure ou bromure de glyoxylyle correspondant ; avec la réaction conduite dans un solvant nitroparaffinique à une température comprise entre -80°C et la température ambiante pendant une période atteignant environ 24 h.

12. Un composé selon les revendications 1, 2, 3, 4, 5, 6, 7 ou 8 à utiliser comme médicament.

13. Utilisation d'un composé selon les revendications 1, 2, 3, 4, 5, 6, 7 ou 8 pour la préparation d'un médicament pour le traitement de la douleur.

14. Une formulation pharmaceutique contenant un composé selon les revendications 1, 2, 3, 4, 5, 6, 7 ou 8 en mélange avec un support pharmaceutiquement acceptable.

15. Utilisation d'un composé selon les revendications 1, 2, 3, 4, 5, 6, 7 ou 8 pour la préparation d'un médicament pour le traitement du vomissement induit par un médicament cytotoxique.

16. Utilisation d'un composé selon les revendications 1, 2, 3, 4, 5, 6, 7 ou 8 pour la préparation d'un médicament pour la thérapie de la migraine soit seul soit en combinaison avec un autre agent antimigraineux.

## Revendications (Revendications pour l'(les) Etat(s) contractant(s) suivant(s): ES)

1. Un procédé de préparation d'un composé de formule : 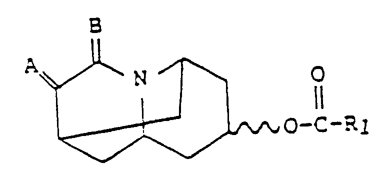 dans laquelle A est =H₂, =O, =(H)(OH) ou =N-OH ; B est =H₂, =(H)(CH₃), =(H)(CH₂NR₃R₄) ou =CH₂, où R₃ et R₄ sont des groupes C₂-C₄ alkyles ou sont combinés pour donner un groupe tétraméthylène, pentaméthylène ou -CH₂CH₂-O-CH₂CH₂- ; R₁ est : 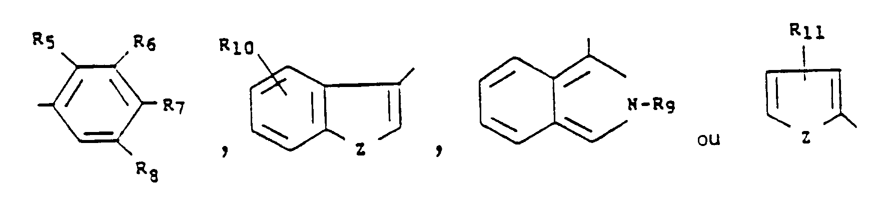 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; le trait ondulé indique que la configuration du substituant oxygène sur le noyau peut être endo ou exo ; et les sels d'addition d'acide et les sels d'ammonium quaternaire pharmaceutiquement acceptables desdits composés, qui comprend la réaction d'un alcool ou d'un dérivé réactif de ce dernier, ledit alcool ayant la formule : 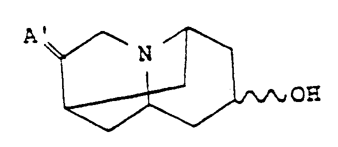 dans laquelle A' est =H₂ ou =O, avec un équivalent réactif d'un acide de formule :
R₁COOH
dans laquelle R₁ est comme défini précédemment, pour donner des composés dans lesquels A est =H₂ ou =O, éventuellement suivie de
(a) la réduction de la cétone produite avec un borohydrure de métal alcalin pour donner les composés dans lesquels A est =(H)(OH), ou bien
(b) la conversion de la cétone produite en un dithiocétal avec l'éthylènedithiol ou le triméthylènedithiol suivie de la réduction par l'hydrazine en présence du nickel Raney pour donner les composés dans lesquels A est =H₂, ou bien
(c) la réaction de la cétone produite avec le chlorhydrate d'hydroxylamine pour donner les composés dans lesquels A est =N-OH, ou bien
(d) la réaction de la cétone produite avec le formaldéhyde et une amine secondaire appropriée pour donner les composés dans lesquels B est =(H)(CH₂NR₃R₄), suivie, lorsque B est un groupe diméthylaminométhyle, d'un chauffage pour donner les composés dans lesquels B est =CH₂, puis suivie de l'hydrogénation pour donner les composés dans lesquels B est =(H)(CH₃).

2. Un procédé selon la revendication 1 pour la préparation d'un composé de formule : 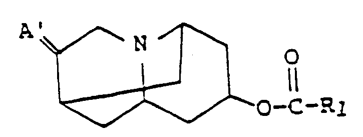 dans laquelle A' est =H₂ ou= O ; R₁ est : 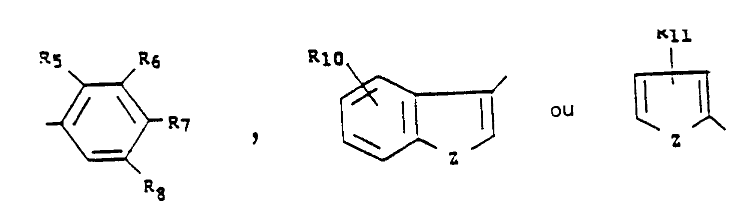 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle; et les sels d'addition d'acide et les sels d'ammonium quaternaire pharmaceutiquement acceptables desdits composés, qui comprend la réaction d'un alcool ou d'un dérivé réactif de ce dernier, ledit alcool ayant la formule : 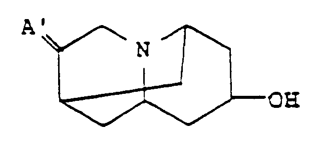 dans laquelle A' est défini comme précédemment, avec un équivalent réactif d'un acide de formule :
R₁COOH
dans laquelle R₁ est défini comme précédemment.

3. Un procédé selon la revendication 1 pour préparer un composé de formule : 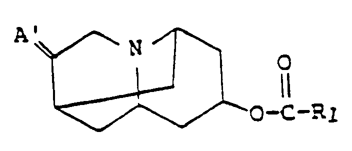 dans laquelle A' est =H₂ ou =O ; R₁ est : 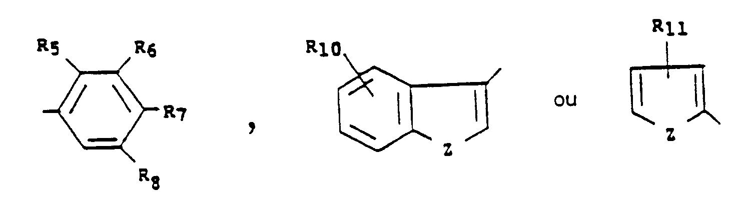 où R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; Z est NR₉, O ou S ; R₉ est un atome d'hydrogène, un groupe C₁-C₄ alkyle ou phényle (C₁-C₂ alkyle) ; et les sels d'addition d'acide et les sels d'ammonium quaternaire pharmaceutiquement acceptables desdits composés, qui comprend la réaction d'un alcool de formule : 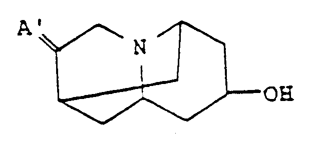 avec un acide de formule :
R₁COOH
dans laquelle A', R₁ sont définis comme précédemment ; ledit alcool étant utilisé sous la forme d'un sel de superacide de l'alcool et en présence d'un équivalent d'un sel de métal lourd du même superacide ; ledit acide étant utilisé sous la forme du chlorure ou du bromure d'acide correspondant ou du chlorure ou bromure de glyoxylyle correspondant ; avec la réaction conduite dans un solvant nitroparaffinique à une température comprise entre -80°C et la température ambiante pendant une période atteignant environ 24h.

4. Un procédé selon la revendication 1 pour la préparation d'un composé qui a la formule : 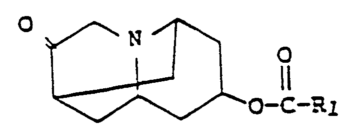 dans laquelle R₁ est 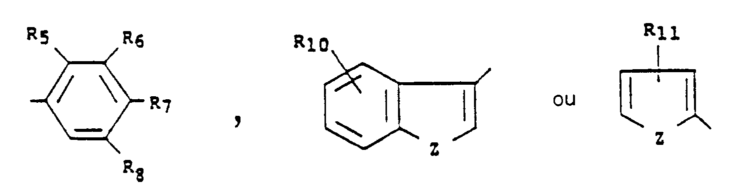 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; et les sels d'addition d'acide et les sels d'ammonium quaternaire pharmaceutiquement acceptables desdits composés, qui comprend la réaction d'un alcool de formule : 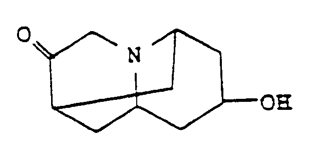 avec un acide de formule :
R₁COOH
dans laquelle R₁ est défini comme ci-dessus ; ledit alcool étant utilisé sous la forme d'un sel de superacide de l'alcool et en présence d'un équivalent d'un sel de métal lourd du même superacide ; cet acide étant utilisé sous la forme de son chlorure d'acide ou son bromure d'acide correspondant ou du chlorure ou du bromure de glyoxylyle correspondant ; avec la réaction conduite dans un solvant nitroparaffinique à une température comprise entre -80°C et la température ambiante pendant une période atteignant environ 24h.

5. Un procédé selon la revendication 1 pour la préparation d'un composé qui a la formule : 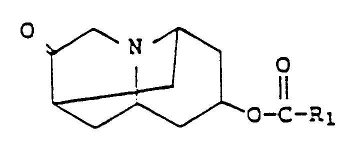 dans laquelle R₁ est 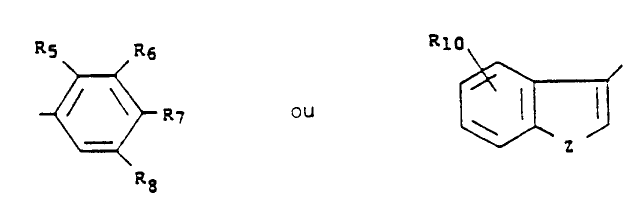 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂, et les sels d'addition d'acide et les sels d'ammonium quaternaire pharmaceutiquement acceptables desdits composés, qui comprend la réaction d'un alcool de formule : 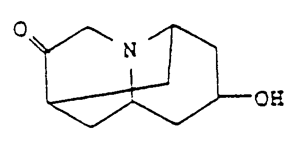 avec un acide de formule :
R₁COOH
où R₁ est défini comme ci-dessus ; ledit alcool étant utilisé sous la forme d'un sel de superacide de l'alcool et en présence d'un équivalent d'un sel de métal lourd du même superacide ; ledit acide étant utilisé sous la forme du chlorure ou du bromure d'acide correspondant ou du chlorure ou du bromure de glyoxylyle correpondant ; avec la réaction conduite dans un solvant nitroparaffinique à une température comprise entre -80°C et la température ambiante pendant une période atteignant environ 24 h.

6. Un procédé selon la revendication 1 pour la préparation de l'endo-8-(3,5-diméthylbenzoyloxy)hexahydro-2,6-méthano-2H-quinolizine-3(4H)-one qui comprend la réaction de l'endo-hexahydro-8-hydroxy-2,6-méthano-2H-quinolizine-3(4H)-one d'abord avec l'acide hydrofluoroborique, puis avec le tétrafluoroborate d'argent et finalement avec le chlorure de 3,5-diméthylbenzoyle.

7. Un procédé selon la revendication 1 pour la préparation de l'endo-8-(3-indolylcarbonyloxy)hexahydro-2,6-méthano-2H-quinolizine-3(4H)-one qui comprend la réaction de l'endo-hexahydro-8-hydroxy-2,6-méthano-2H-quinolizine-3(4H)-one d'abord avec l'acide hydrofluoroborique puis avec le tétrafluoroborate d'argent et finalement avec le chlorure d'acide indole-3-carboxylique.

8. Un procédé selon la revendication 1 pour la préparation de l'endo-hexahydro-8-hydroxy-2,6-méthano-2H-quinolizine-3(4H)-one.

9. Un procédé de préparation d'une composition pharmaceutique comprenant la combinaison d'un composé préparé selon l'une quelconque des revendications 1 à 8 avec un support pharmaceutiquement acceptable.

10. Un procédé selon la revendication 9, selon laquelle la composition pharmaceutique préparée est utile pour le traitement de la douleur.

11. Un procédé selon la revendication 9, selon laquelle la composition pharmaceutique préparée est utile dans le traitement du vomissement induit par un médicament cytotoxique.

12. Un procédé selon la revendication 9, selon laquelle la composition pharmaceutique préparée est utile dans la thérapie de la migraine soit seule soit en combinaison avec un autre agent antimigraineux.

## Revendications (Revendications pour l'(les) Etat(s) contractant(s) suivant(s): GR)

1. Un composé de formule : 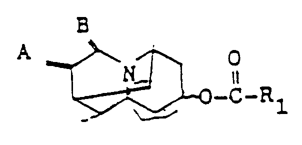 dans laquelle A est =H₂, =O, =(H)(OH) ou =N-OH ; B est =H₂, =(H)(CH₃), =(H)(CH₂NR₃R₄) ou =CH₂, où R₃ et R₄ sont des groupes C₂-C₄ alkyles ou sont combinés pour donner un groupe tétraméthylène, pentaméthylène ou -CH₂CH₂-O-CH₂CH₂- ; R₁ est : 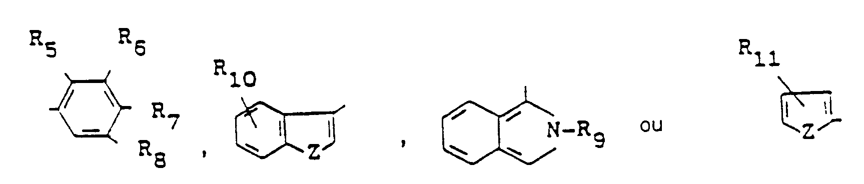 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; le trait ondulé indique que la configuration du substituant oxygène sur le noyau peut être endo ou exo ; et les sels d'addition d'acide et les sels d'ammonium quaternaire pharmaceutiquement acceptables desdits composés.

2. Un composé selon la revendication 1 qui a la formule : 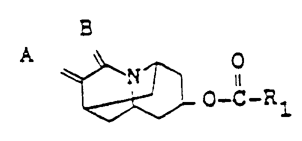 dans laquelle A est =H₂, =O, =(H)(OH) ou =N-OH ; B est =H₂, =(H)(CH₃), =(H(CH₂NR₃R₄) ou =CH₂, dans laquelle R₃ et R₄ sont des groupes C₂-C₄ alkyle ou sont combinés pour donner un groupe tétraméthylène, pentaméthylène ou -CH₂CH₂-O-CH₂CH₂- ; R₁ est : 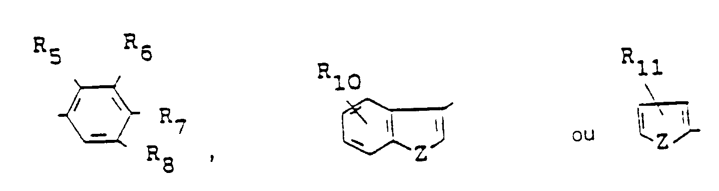 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle, et les sels d'addition d'acide et les sels d'ammonium quaternaire pharmaceutiquement acceptables desdits composés.

3. Un composé selon la revendication 1 qui a la formule : 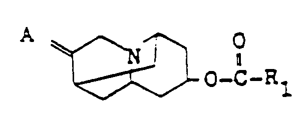 dans laquelle A est =H₂, =O, =(H)(OH) ou =N-OH; R₁ est : 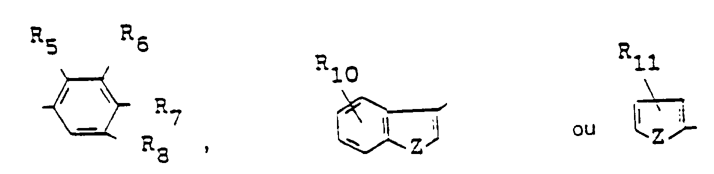 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényle (C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle, et les sels d'addition d'acide ou les sels d'ammonium quaternaire pharmaceutiquement acceptables desdits composés.

4. Un composé selon la revendication 1 qui a la formule : 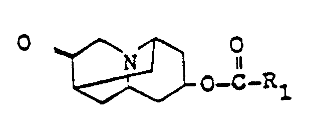 dans laquelle R₁ est : 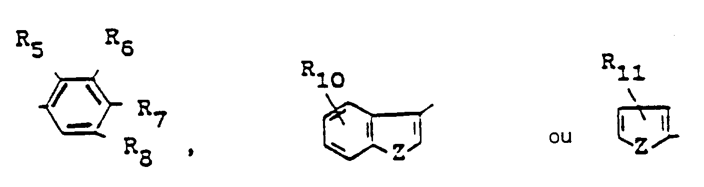 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyl ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle, et les sels d'addition d'acide et les sels d'ammonium quaternaire pharmaceutiquement acceptables desdits composés.

5. Un composé selon la revendication 1 qui a la formule : 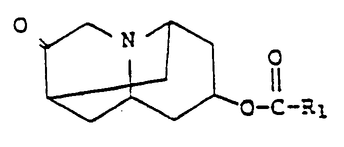 dans laquelle R₁ est : 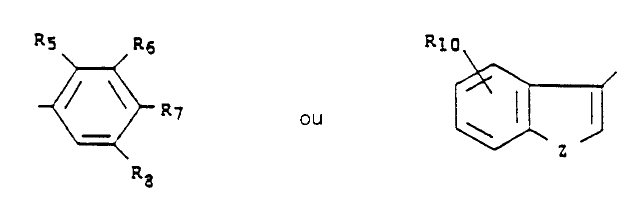 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂, et les sels d'addition d'acide et les sels d'ammonium quaternaire pharmaceutiquement acceptables desdits composés.

6. Un composé selon la revendication 1 qui est l'endo-8-(3,5-diméthylbenzyloxy)hexahydro-2,6-méthano-2H-quinolizine-3(4H)-one.

7. Un composé selon la revendication 1 qui est l'endo-8-(3-indolylcarbonyloxy)hexahydro-2,6-méthano-2H-quinolizine-3(4H)-one.

8. Un composé selon la revendication 1 qui est l'endo-hexahydro-8-hydroxy-2,6-méthano-2H-quinolizine-3(4H)-one.

9. Un procédé de préparation d'un composé selon la revendication 1 qui comprend la réaction d'un alcool ou d'un dérivé réactif de ce dernier, ledit alcool ayant la formule : 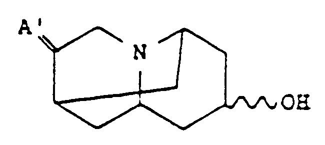 dans laquelle A' est =H₂ ou =O, avec un équivalent réactif d'un acide de formule :
R₁COOH
dans laquelle R₁ est comme défini dans la revendication 1, pour donner des composés dans lesquels A est =H₂ ou =O, éventuellement suivie de
(a) la réduction de la cétone produite avec un borohydrure de métal alcalin pour donner les composés dans lesquels A est =(H)(OH), ou bien
(b) la conversion de la cétone produite en un dithiocétal avec l'éthylènedithiol ou le triméthylènedithiol suivie de la réduction par l'hydrazine en présence du nickel Raney pour donner les composés dans lesquels A est =H₂, ou bien
(c) la réaction de la cétone produite avec le chlorhydrate d'hydroxylamine pour donner les composés dans lesquels A est =N-OH, ou bien
(d) la réaction de la cétone produite avec le formaldéhyde et une amine secondaire appropriée pour donner les composés dans lesquels B est =(H)(CH₂NR₃R₄), suivie, lorsque B est un groupe diméthylaminométhyle, d'un chauffage pour donner les composés dans lesquels B est =CH₂, puis suivie de l'hydrogénation pour donner les composés dans lesquels B est =(H)(CH₃).

10. Un procédé de préparation d'un composé de formule : 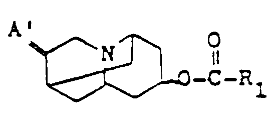 dans laquelle A' est =H₂ =O; R₁ est : 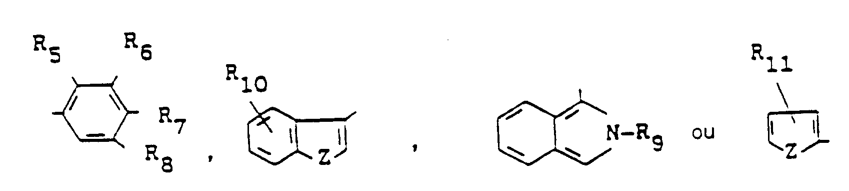 où Z est NR₉, O ou S ; R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; le trait ondulé indique que la configuration du substituant oxygène sur le noyau peut être endo ou exo ; et les sels d'addition d'acide et les sels d'ammonium quaternaire pharmaceutiquement acceptables desdits composés, qui comprend la réaction d'un alcool ou d'un dérivé réactif de ce dernier, ledit alcool ayant la formule : 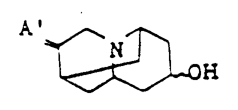 dans laquelle A' est défini conune précédemment, avec un équivalent réactif d'un acide de formule :
R₁COOH
dans laquelle R₁ est défini comme précédemment.

11. Un procédé selon la revendication 9 pour préparer un composé de formule : 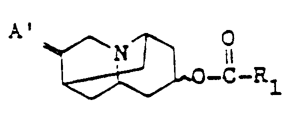 dans laquelle A' est =H₂ ou =O ; R₁ est : 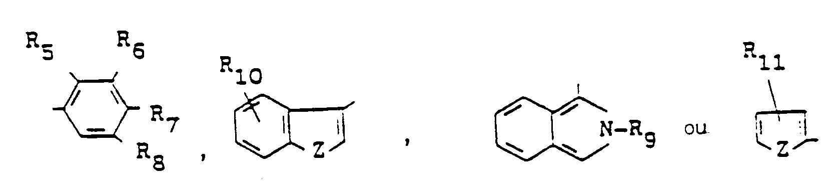 où R₅, R₆ et R₈ désignent chacun un atome d'hydrogène, d'halogène, un groupe C₁-C₃ alkyle ou C₁-C₃ alcoxy ; R₇ est un atome d'hydrogène, un groupe amino, (C₁-C₄ alkyl)amino, (C₁-C₄ alkyl)₂amino ou nitro ; R₉ est un atome d'hydrogène, un groupe C₁-C₄ alkyle ou phényl(C₁-C₂ alkyle) ; R₁₀ est un atome d'hydrogène, d'halogène, un groupe C₁-C₄ alkyle, C₁-C₄ alcoxy, hydroxy, cyano ou -CONH₂ ; R₁₁ est un atome d'hydrogène, d'halogène, un groupe C₁-C₄ alkyle ou phényle ; Z est NR₉, O ou S ; la configuration du substituant oxygène sur le noyau comme indiqué par le trait ondulé est endo ; et les sels d'addition d'acide et les sels d'ammonium quaternaire pharmaceutiquement acceptables desdits composés, qui comprend la réaction d'un alcool de formule : 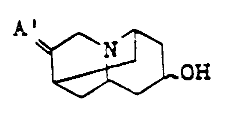 avec un acide de formule :
R₁COOH
dans laquelle A', R₁ et le trait ondulé sont définis comme précédemment ; ledit alcool étant utilisé sous la forme d'un sel de superacide de l'alcool et en présence d'un équivalent d'un sel de métal lourd du même superacide ; ledit acide étant utilisé sous la forme du chlorure ou du bromure d'acide correspondant ou du chlorure ou bromure de glyoxylyle correspondant ; avec la réaction conduite dans un solvant nitroparaffinique à une température comprise entre -80°C et la température ambiante pendant une période atteignant environ 24h.

12. Un procédé de préparation d'une composition pharmaceutique comprenant la combinaison d'un composé tel que défini dans l'une quelconque des revendications 1 à 8 avec un support pharmaceutiquement acceptable.

13. Un procédé selon la revendication 12, selon laquelle la composition pharmaceutique est utile pour le traitement de la douleur.

14. Un procédé selon la revendication 12, selon laquelle la composition pharmaceutique est utile dans le traitement du vomissement induit par un médicament cytotoxique.

15. Un procédé selon la revendication 12, selon laquelle la composition pharmaceutique est utile dans la thérapie de la migraine soit seul soit en combinaison avec un autre agent antimigraineux.
